# EUROPEAN PATENT APPLICATION

(11) **EP 4 768 472 A1**
(43) Date of publication of application: **01.07.2026**
(21) Application number: 24853861.3
(22) Date of filing: 15.08.2024
(51) Int. Cl.: C07D 211/58, A61K 9/51, A61K 47/22, A61K 47/24, A61K 48/00, A61P 35/00, A61P 37/02, A61P 3/10, A61P 25/00, A61P 9/00, A61P 3/00

(54) **DRUG DELIVERY MATERIAL AND APPLICATION THEREOF**

(30) Priority: 17.08.2023 CN 202311039860
(71) Applicant: DSCILAB.CO., LTD, Suzhou, Jiangsu 215000 (CN)
(72) Inventor: CUI, Zhiping, Shanghai 201210 (CN); LIU, Xiaohua, Shanghai 201210 (CN); WANG, Songli, Shanghai 201210 (CN); CHEN, Yuanyuan, Shanghai 201210 (CN); SUN, Sijie, Shanghai 201210 (CN)
(74) Representative: Ipsilon
(86) International application number: PCT/CN2024/112388
(87) International publication number: WO 2025/036455

(57) **Abstract**

The present invention relates to a drug delivery material and an application thereof. Specifically provided is a nanoparticle composition comprising a lipid component, the lipid component comprising a compound represented by formula (I). The nanoparticle composition of the present invention is delivered only at the site of administration.

## Description

### TECHNICAL FIELD

This disclosure relates to the field of biomedicine, specifically to a drug delivery material and a use thereof.

### BACKGROUND

Lipid-based materials and their derivatives have been extensively studied for their excellent biocompatibility and good encapsulation efficiency for RNA, making them suitable for in vivo delivery of RNA. RNA is safe and effective, and has promising applications in the development of new therapies for genetic diseases, the expression of functional proteins and antibodies, vaccines, or gene editing. Currently, commercially available lipid delivery vehicles (BNT162b2 of BioNTech and mRNA-1273 of Moderna) exhibit significant expression of target proteins in the liver across various administration routes (intravenous, subcutaneous, and intramuscular injection), posing potential toxic side effects on extrahepatic lesions and hindering clinical translation and application.

Therefore, developing LNP delivery vectors with local expression specificity can provide a novel platform for treating currently intractable or untreatable diseases, greatly expanding the clinical application of RNA therapy.

### SUMMARY

In a first aspect, the present disclosure provides a compound of formula (I) or a salt or isomer thereof, 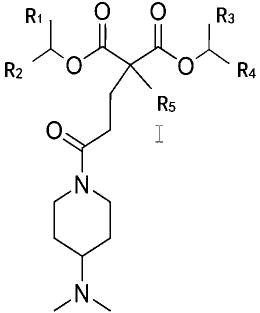 wherein,
R₁ is H or C1-C8 alkyl,
R₂ is C1-10 alkyl or C3-C18 alkadienyl
R₃ is H or R1-R8 alkyl,
R₄ is C1-10 alkyl, or C3-C18 alkadienyl,
R₅ is C1-C14 alkyl group, C2-C14 alkenyl, or 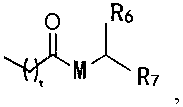
M is O or N,
t is an integer from 3 to 7,
R₆ is H or C1-10 alkyl,
R₇ is C1-10 alkyl, or C3-C18 alkadienyl.

In one or more embodiments, R₁ is the same as R₃, and R₂ is the same as R₄.

In one or more embodiments, R₁ is H, and R₂ is C6-10 alkyl or C14-C18 alkadienyl.

In one or more embodiments, R₁ is C4-C8 alkyl, and R₂ is C4-C8 alkyl.

In one or more embodiments, R₃ is H, and R₄ is C6-10 alkyl or C14-C18 alkadienyl.

In one or more embodiments, R₃ is C4-C8 alkyl, and R₄ is C4-C8 alkyl.

In one or more embodiments, R₅ is C1-C4 alkyl, C10-C14 alkyl, C10-C14 alkenyl, or 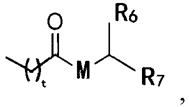
M is O or N,
t is 3 or 7,
R₆ is C4-C8 alkyl and R₇ is C4-C8 alkyl, or R₆ is H and R₇ is C14-C18 alkadienyl.

In one or more embodiments, R₁ is H, R₂ is C6-10 alkyl, R₁ is the same as R₃, R₂ is the same as R₄, R₅ is C10-C14 alkyl, C10-C14 alkenyl, or 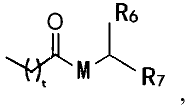 M is O or N, t is 7, R₆ is C4-C8 alkyl, and R₇ is C4-C8 alkyl.

In one or more embodiments, R₁ is H, R₂ is a C14-C18 alkadienyl, R₁ is the same as R₃, R₂ is the same as R₄, R₅ is 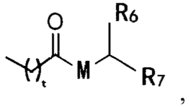 M is O, t is 3, R₆ is H, and R₇ is a C14-C18 alkadienyl. Preferably, R₇ is the same as R₂.

In one or more embodiments, R₁ is a C4-C8 alkyl, R₂ is a C4-C8 alkyl, R₁ is the same as R₃, R₂ is the same as R₄, R₅ is a C1-C4 alkyl group, 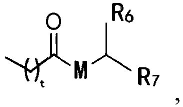 M is O, t is 3, R₆ is H, and R₇ is C14-C18 alkadienyl.

In one or more embodiments, each of the alkyl is n-alkyl.

In one or more embodiments, when R₂, R₄, and R₇ are C14-C18 alkadienyl, the alkenyls of the C14-C18 alkadienyls are located on any two carbons from carbons 7 to 14. In one or more embodiments, the alkenyls are located on carbons P and P+3, where P is 7 to 11. In one or more embodiments, the C14-C18 alkadienyl is 9, 12- alkadienyl with 14 to 18 carbons.

In one or more embodiments, the compound of formula (I) is as described in any one of formulae 15, 15A-F.

The present disclosure further provides a nanoparticle composition comprising a lipid component, wherein the lipid component comprises a compound as described in any one of the embodiments herein (e.g., a compound of formula (I)).

In one or more embodiments, the lipid component further comprises phospholipids (such as polyunsaturated lipids), PEG lipids, and structured lipids.

In one or more embodiments, the nanoparticle composition further comprises a therapeutic agent and/or a prophylactic agent.

The present disclosure further provides a pharmaceutical composition comprising the nanoparticle composition described in any one of the embodiments herein and a pharmaceutically acceptable adjuvant. The pharmaceutical composition is a locally acting pharmaceutical composition.

The present disclosure further provides a method for delivering a therapeutic agent and/or a prophylactic agent to cells, comprising the step of administering (e.g., injecting) a nanoparticle composition to a subject, wherein the composition comprises: (1) a lipid component, including phospholipids (e.g., polyunsaturated lipids), PEG lipids, structured lipids, and compounds of formula (I), and (2) a therapeutic agent and/or a prophylactic agent, wherein the administering comprises contacting the cells with the nanoparticle composition, thereby delivering the therapeutic agent and/or prophylactic agent to the cells.

In one or more embodiments, the therapeutic agent and/or prophylactic agent is a polynucleotide.

In one or more embodiments, the therapeutic agent and/or prophylactic agent is mRNA.

In one or more embodiments, the cells are mammalian cells.

In one or more embodiments, the delivery is local (rather than systemic) delivery.

In one or more embodiments, the cells are cells in the vicinity of the administration site.

In one or more embodiments, the cells are cells of the same organ or tissue at the administration site. The organ or tissue is, for example, heart, liver, spleen, lung, muscle, or femur.

In one or more embodiments, the subject is a mammal, such as a human.

The present disclosure further provides a method for producing a polypeptide in a cell, comprising the step of contacting the cell with a nanoparticle composition, wherein the composition comprises: (1) a lipid component, including phospholipids (such as polyunsaturated lipids), PEG lipids, structured lipids, and a compound of formula (I) or a salt or isomer thereof as described in any one of the embodiments herein, and (2) an mRNA encoding the polypeptide, whereby the mRNA can be translated in the cell to produce the polypeptide.

In one or more embodiments, the cells are mammalian cells.

The present disclosure further provides a method for treating a disease or disorder in a mammal (e.g., a human) or for achieving local efficacy of a therapeutic agent and/or prophylactic agent by administering the therapeutic agent and/or prophylactic agent locally (rather than systemically), comprising the step of locally administering (e.g., injecting) a therapeutically effective amount of a nanoparticle composition to the mammal, wherein the nanoparticle composition comprising: (1) a lipid component comprising phospholipids (e.g., polyunsaturated lipids), PEG lipids, structural lipids, and a compound of formula (I), or a salt or isomer thereof, as described in any one of the embodiments herein; and (2) a therapeutic agent and/or prophylactic agent (e.g., mRNA or siRNA).

In one or more embodiments, the disease or disorder is a disease or disorder characterized by dysfunction or abnormality of a protein or polypeptide.

In one or more embodiments, the disease or disorder is selected from the group consisting of rare diseases, infectious diseases, cancers and proliferative diseases, genetic diseases (such as cystic fibrosis), autoimmune diseases, diabetes, neurodegenerative diseases, cardiovascular diseases, renovascular diseases, and metabolic diseases.

The present disclosure further provides a method for delivering a therapeutic agent and/or prophylactic agent to a local region (non-systemicity) of a mammal, comprising the step of administering a nanoparticle composition to a subject (e.g., a mammal), wherein the composition comprises: (1) a lipid component comprising phospholipids, PEG lipids, structural lipids, and a compound of formula (I), or a salt or isomer thereof, as described in any one of the embodiments herein, and (2) a therapeutic agent and/or prophylactic agent (e.g., mRNA or siRNA), wherein the administration comprises contacting mammalian cells with the nanoparticle composition, thereby delivering the therapeutic agent and/or prophylactic agent to the local region of the mammal.

In one or more embodiments, the local region is a target organ or tissue, e.g., heart, liver, spleen, lung, muscle or femur.

The present disclosure further provides a method for preparing the compound of formula (I) described herein or a method for preparing the nanoparticle composition described herein.

Use of a compound of formula (I), or a salt or isomer thereof, as described in any one of the embodiments herein, or a nanoparticle composition as described in any one of the embodiments herein, in the preparation of a locally acting medicament for treating or preventing a disease or disorder.

In one or more embodiments, the disease or disorder is one that benefits from a therapeutic agent and/or prophylactic agent for local (rather than systemic) delivery.

In one or more embodiments, the medicament is a therapeutic agent and/or prophylactic agent for local (rather than systemic) delivery.

In one or more embodiments, the nanoparticle composition or a nanoparticle composition formed from the compound of formula (I) is used for delivering a therapeutic agent and/or prophylactic agent to a local region (non-systemicity).

In one or more embodiments, the local region is a target organ or tissue, e.g., heart, liver, spleen, lung, muscle or femur.

### DESCRIPTION OF DRAWINGS

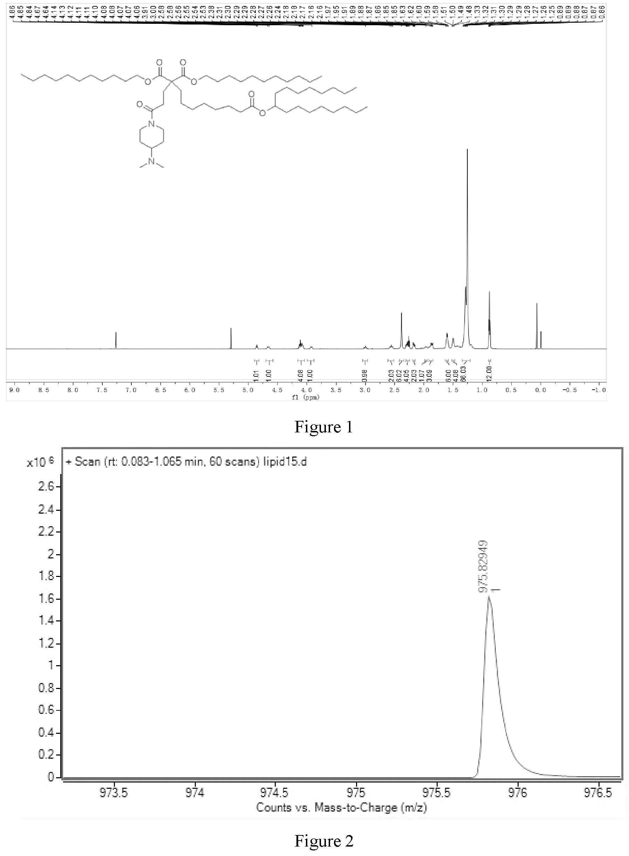

Figure 1 shows the nuclear magnetic resonance hydrogen spectrum of compound 15.
Figure 2 shows the mass spectrum of compound 15.

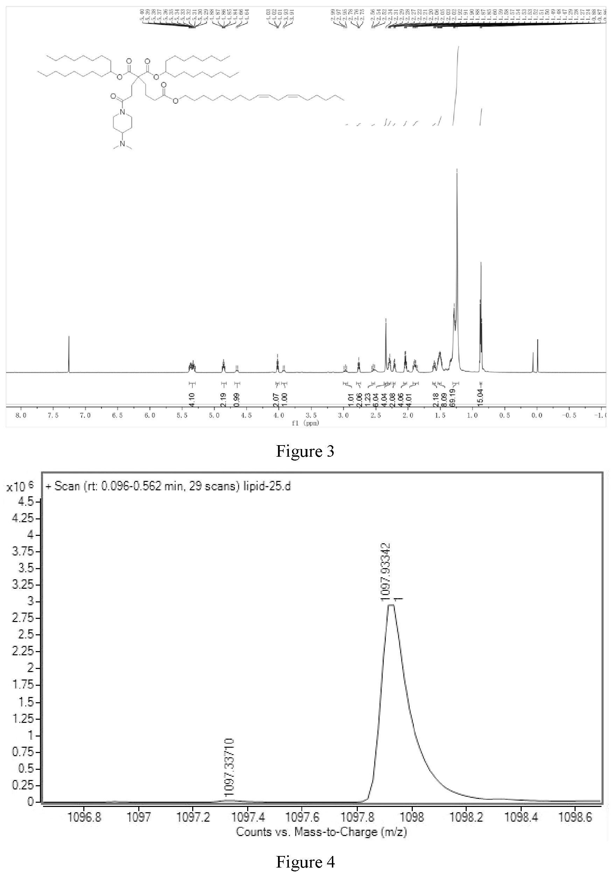

Figure 3 shows the nuclear magnetic resonance hydrogen spectrum of compound 15A.
Figure 4 shows the mass spectrum of compound 15A.

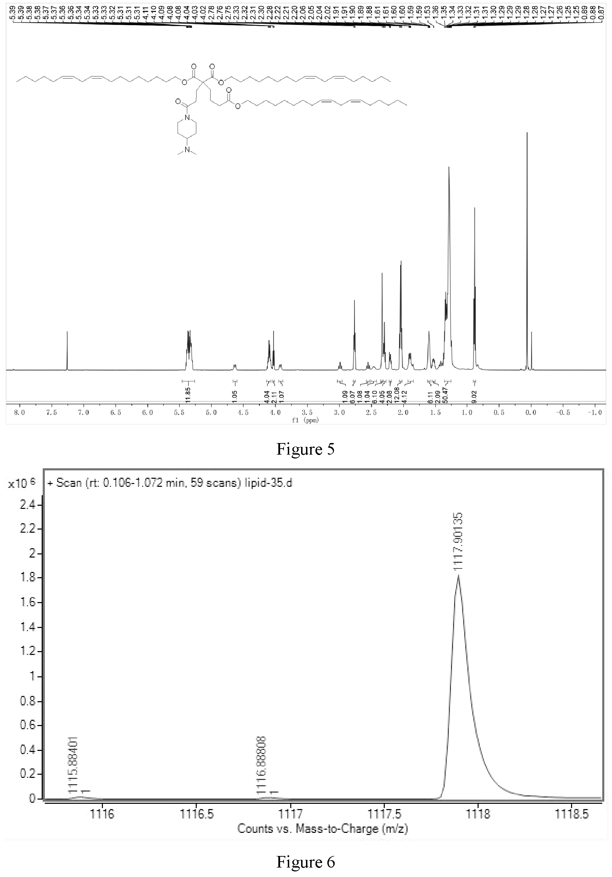

Figure 5 shows the nuclear magnetic resonance hydrogen spectrum of compound 15B.
Figure 6 shows the mass spectrum of compound 15B.

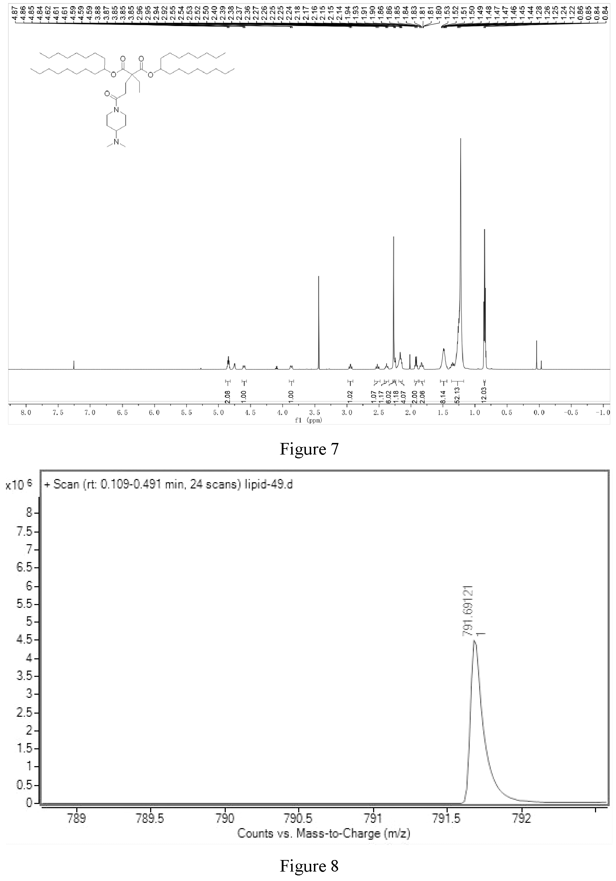

Figure 7 shows the nuclear magnetic resonance hydrogen spectrum of compound 15C.
Figure 8 shows the mass spectrum of compound 15C.
Figure 9 shows the particle size distribution of LNPs. SM-102 LNP-1 has a particle size of 92.38±1.639 nm and a PDI of 0.035±0.022; SM-102 LNP-2 has a particle size of 68.53±1.003 nm and a PDI of 0.053±0.027; 15 LNP-1 has a particle size of 87.34±2.507 nm and a PDI of 0.054±0.017; 15 LNP-2 has a particle size of 64.42±0.7681 nm and a PDI of 0.107±0.037; 15A LNP has a particle size of 73.84±0.94 nm and a PDI of 0.036±0.028; 15B LNP has a particle size of 74.50±2.22 nm and a PDI of 0.23±0.02; 15C LNP has a particle size of 64.57±1.26 nm and a PDI of 0.11±0.04.
Figure 10 shows the semi-quantitative fluorescence results of in vivo small animal imaging of C57 mice after gastrocnemius muscle injection of LNPs. SM-102 LNP-2 shows high fluorescence intensity values at both the intramuscular injection site and the liver site, while 15LNP-2 only shows high fluorescence intensity values at the intramuscular injection site, with no fluorescence intensity values observed at the liver site, indicating better local expression performance of 15LNP-2.
Figure 11 shows the expression results in C57 mice after intramuscular injection of 15 LNP-2. 15LNP-2 was only significantly expressed at the intramuscular injection site, with no expression observed in the liver, indicating its good local expression performance.
Figure 12 shows the changes in expression activity over time and the local expression area following intramuscular injection of 15 LNP-2 in C57 mice. After intramuscular injection of 15 LNP-2, the expression area decreased with increasing time, and the expression site was confined only to the injection site throughout the experimental period.
Figure 13 shows the changes in expression activity over time and the local expression area following intramuscular injection of 15 LNP-2 in SD rats. After intramuscular injection of 15 LNP-2, the expression area decreased with increasing time, and the expression site was confined only to the injection site throughout the experimental period.
Figure 14 shows the local injection expression results of SM-102 LNP-2. Expression of SM-102 LNP-2 was observed at both the intramuscular injection site and the liver site, indicating that SM-102 LNP-2 does not possess local expression capability.
Figure 15 shows the local injection expression results of 15A LNP, 15B LNP and 15C LNP. Expression of 15A LNP was observed at both the intramuscular injection site and the liver site, indicating that 15A LNP does not possess local expression capability; the expression efficacy of 15B LNP at the intramuscular injection site was weak; 15C LNP exhibited expression only at the intramuscular injection site, indicating that 15C LNP has favorable local expression capability.
Figure 16 shows the semi-quantitative fluorescence results of in vivo small animal imaging following myocardial injection of LNPs in SD rats. SM-102 LNP-1, SM-102 LNP-2 and 15 LNP-1 exhibited high fluorescence intensity values at both the myocardial injection site and the liver site; 15 LNP-2 showed high fluorescence intensity values only at the myocardial injection site with low fluorescence intensity values at the liver site, indicating that 15 LNP-2 has favorable local expression capability.
Figure 17 shows the expression results of SM-102 LNP-1 following myocardial injection. Expression of SM-102 LNP-1 was observed at both the myocardial injection site and the liver site, indicating that SM-102 LNP-1 does not possess local expression capability.
Figure 18 shows the expression results of SM-102 LNP-2 following myocardial injection. Expression of SM-102 LNP-2 was observed at both the myocardial injection site and the liver site, indicating that SM-102 LNP-2 does not possess local expression capability.
Figure 19 shows the expression results of 15 LNP-1 following myocardial injection. Expression of 15 LNP-1 was observed at both the myocardial injection site and the liver site, indicating that 15 LNP-1 does not possess local expression capability.
Figure 20 shows the expression results of 15 LNP-2 following myocardial injection. 15 LNP-2 exhibited significant expression only at the myocardial injection site, with no expression observed at the liver site, indicating that 15 LNP-2 has favorable local expression capability.
Figure 21 shows the expression results of 15 LNP-2 following splenic injection in C57 mice. 15 LNP-2 exhibited significant expression only at the splenic injection site, with no expression observed at other sites, indicating that 15 LNP-2 has favorable local expression capability.
Figure 22 shows the expression results of 15 LNP-2 following renal injection in C57 mice. 15 LNP-2 exhibited significant expression only at the renal injection site, with no expression observed at other sites, indicating that 15 LNP-2 has favorable local expression capability.
Figure 23 shows the expression results of 15 LNP-2 following splenic injection in SD rats. 15 LNP-2 exhibited significant expression only at the splenic injection site, with no expression observed at other sites, indicating that 15 LNP-2 has favorable local expression capability.
Figure 24 shows the expression results of 15 LNP-2 following renal injection in SD rats. 15 LNP-2 exhibited significant expression only at the renal injection site, with no expression observed at other sites, indicating that 15 LNP-2 has favorable local expression capability.

### DETAILED DESCRIPTION

The inventor synthesized a novel ionizable cationic lipid and utilized it to prepare LNP. When the LNP loaded with mRNA was injected into the leg muscle, heart muscle, spleen, and kidney of mice, in vivo fluorescence imaging of the small animals revealed that the delivery system could only express mRNA-encoded proteins at the injection site, with no expression observed in other major organs (heart, liver, spleen, lung, and kidney).

The disclosure relates to novel lipids and lipid nanoparticle compositions comprising novel lipids. The disclosure further provides methods for delivering therapeutic and/or prophylactic agents to mammalian cells, particularly to a local region of a mammal, producing a desired polypeptide in local mammalian cells, and for treating diseases or disorders in mammals.

The method for delivering therapeutic and/or prophylactic agents to mammalian cells or local regions involves administering a nanoparticle composition comprising the therapeutic and/or prophylactic agent to a subject, wherein the administration involves contacting the cells or local regions with the composition, thereby the therapeutic and/or prophylactic drug is delivered to the cells or local regions.

Herein, the term "local" refers to cells, tissues or organs in a certain range of the administration site. In the prior art, special dosage forms are generally required to retain drugs at the administration site, such as creams, ointments, sprays and powder aerosols, which are mostly applied to the body surface or mucosal surface. However, the inventors have found that the drugs (e.g., the nanoparticle compositions or pharmaceutical compositions described herein) prepared by using the compounds of formula (I) described herein can achieve local drug delivery and local efficacy in organs, tissues or cells. In some embodiments, the nanoparticle compositions of the present disclosure are suitable for the preparation of locally applied, locally acting products (locally applied, locally acting products, LALAP). In the present disclosure, the terms "locally acting drug", "locally acting pharmaceutical composition", " locally applied, locally acting products" or " locally applied, locally acting" refer to drugs that are applied locally and exert their effects at the applied site. If such drugs produce systemic effects (e.g., liver-targeting effects), the effects shall be deemed as unintended pharmacological effects. A local region may include only cells, only organs (e.g., heart, liver, spleen, kidney or lung) or only tissues (e.g., muscle, skin or bone). In some embodiments of the present disclosure, the local region refers to only the cells, tissues or organs at the administration site.In some embodiments, when local administration is performed to an organ (e.g., heart, liver, spleen, kidney or lung), the drug is delivered only to the interior of the target organ at the administration site; when local administration is performed to a tissue (e.g., a specific muscle), the drug is delivered only to the interior of the target tissue (e.g., the specific muscle) at the administration site; when local administration is performed to cells, the drug is delivered only to the cells in the organ or tissue (e.g., the muscle where the said cells are located) at the administration site. In some embodiments, when administration is performed to the gastrocnemius muscle, the drug is delivered only to the interior of the gastrocnemius muscle.

In some embodiments, "local" may refer to the LNP composition, therapeutic agent, and/or prophylactic agent (mRNA) being primarily maintained in an organ (such as the heart, liver, spleen, kidney, or lung) in 4-8 hours (e.g., 6 hours) after administration of the LNP composition described herein to the organ. In some embodiments, "local" may refer to the LNP composition, therapeutic agent, and/or prophylactic agent (mRNA) being primarily maintained in a tissue (such as muscle or bone) in 4-8 hours (e.g., 4 hours) after administration of the LNP composition described herein to the tissue; specifically, not being delivered to the liver in 4-8 hours (e.g., 4 hours) after administration.

### Lipid and lipid nanoparticle composition

The lipids described herein can be advantageously utilized in lipid nanoparticle compositions to deliver therapeutic and/or prophylactic agents to local regions of mammals. The lipids described herein exhibit little or no immunogenicity.

In some aspects, the compounds described herein have the formula (I) or a salt or isomer thereof. The lipid according to formula (I) may have positive charge or partial positive charge at physiological pH. Such lipids may be referred to as cationic lipids.

As used herein, the term "alkyl" or "alkyl group" refers to a straight or branched chain saturated hydrocarbon containing one or more carbon atoms (e.g., 1, 2, 3, 4, 5, 6, 7, 8, 9, 10, 11, 12, 13, 14, 15, 16, 17, 18, 19, 20, or more carbon atoms), which may be optionally substituted. The notation "C1-14 alkyl" refers to an optionally substituted straight or branched chain saturated hydrocarbon containing 1-14 carbon atoms. Unless otherwise specified, the alkyl described herein refer to both unsubstituted and substituted alkyl.

As used herein, the term "alkenyl" or "alkenyl" refers to a straight-chain or branched hydrocarbon comprising two or more carbon atoms (e.g., 2, 3, 4, 5, 6, 7, 8, 9, 10, 11, 12, 13, 14, 15, 16, 17, 18, 19, 20 or more carbon atoms) and at least one carbon-carbon double bond, which is optionally substituted. The notation "C2-14 alkenyl" refers to an optionally substituted straight-chain or branched hydrocarbon containing 2-14 carbon atoms and at least one carbon-carbon double bond. Alkenyl may include 1, 2, 3, 4 or more carbon-carbon double bonds. Unless otherwise specified, alkenyl as described herein refers to both unsubstituted and substituted alkenyl.

As used herein, the term "alkynyl"or "alkynyl group " refers to a straight or branched-chain hydrocarbon comprising two or more carbon atoms (e.g., 2, 3, 4, 5, 6, 7, 8, 9, 10, 11, 12, 13, 14, 15, 16, 17, 18, 19, 20 or more carbon atoms) and at least one carbon-carbon triple bond, which is optionally substituted. The symbol "C2-14 alkynyl" refers to an optionally substituted straight or branched-chain hydrocarbon comprising 2-14 carbon atoms and at least one carbon-carbon triple bond. An alkynyl may contain 1, 2, 3, 5 or more carbon-carbon triple bonds. Unless otherwise specified, the alkynyl described herein refer to both unsubstituted and substituted alkynyl.

As used herein, the term "carbocycle" or "carbocyclic group" refers to an optionally substituted monocyclic or polycyclic system comprising one or more rings of carbon atoms. The ring may be a 3-, 4-, 5-, 6-, 7-, 8-, 9-, 10-, 11-, 12-, 13-, 14-, 15-, 16-, 17-, 18-, 19-, 20-or more-membered ring. The symbol "C3-6 carbocycle" refers to a carbocycle consisting of a monocyclic ring containing 3 to 6 carbon atoms. A carbocycle may comprise one or more carbon-carbon double bonds or triple bonds and can be non-aromatic or aromatic (e.g., cycloalkyl or aryl). Examples of carbocycles include cyclopropyl, cyclopentyl, cyclohexyl, phenyl and naphthyl.

As used herein, the term "cycloalkyl" refers to a non-aromatic carbocycle which may or may not comprise any double bonds or triple bonds. Unless otherwise specified, the carbocycles described herein refer to both unsubstituted and substituted carbocyclic groups, i.e., optionally substituted carbocycles.

As used herein, the term "heterocycle" or "heterocyclic group" refers to an optionally substituted monocyclic or polycyclic system comprising one or more rings, wherein at least one ring comprises at least one heteroatom. The heteroatom may be, for example, a nitrogen, oxygen or sulfur atom. The ring may be a 3-, 4-, 5-, 6-, 7-, 8-, 9-, 10-, 11-, 12-, 13-, 14- or more-membered ring. A heterocycle may comprise one or more double bonds or triple bonds, and can be non-aromatic or aromatic (e.g., heterocycloalkyl or heteroaryl). Examples of heterocycles comprise imidazolyl, imidazolidinyl, oxazolyl, oxazolidinyl, thiazolyl, thiazolidinyl, pyrazolidinyl, pyrazolyl, isoxazolidinyl, isoxazolyl, isothiazolidinyl, isothiazolyl, morpholinyl, pyrrolyl, pyrrolidinyl, furyl, tetrahydrofuryl, thienyl, pyridyl, piperidinylalkynyl, quinolinyl and isoquinolinyl.

As used herein, the term "heterocycloalkyl" refers to a non-aromatic heterocycle which may or may not comprise any double bonds or triple bonds. Unless otherwise specified, the heterocycles described herein refer to both unsubstituted and substituted heterocyclic groups, i.e., optionally substituted heterocycles.

As used herein, the term "aryl" refers to an optionally substituted carbocyclic group comprising one or more aromatic rings. Examples of aryl groups comprise phenyl and naphthyl.

As used herein, the term "heteroaryl" refers to an optionally substituted heterocyclic group comprising one or more aromatic rings. Examples of heteroaryl groups comprise pyrrolyl, furyl, thienyl, imidazolyl, oxazolyl and thiazolyl. Both aryl and heteroaryl groups may be optionally substituted. Unless otherwise specified, the aryl or heteroaryl groups described herein refer to both unsubstituted and substituted groups, i.e., optionally substituted aryl or heteroaryl groups.

Unless otherwise specified, alkyl, alkenyl and cyclic groups (e.g., carbocyclic groups and heterocyclic groups) may be optionally substituted. Optional substituents may be selected from, but are not limited to, halogen atoms (e.g., chloro, bromo, fluoro or iodo groups), carboxylic acids (e.g., -C(O)OH), alcohols (e.g., hydroxyl groups, -OH), esters (e.g., -C(O)OR or -OC(O)R), aldehydes (e.g., -C(O)H), carbonyl groups (e.g., -C(O)R, or represented by C=O), acyl halides (e.g., -C(O)X, where X is a halide selected from bromide, fluoride, chloride and iodide), carbonates (e.g., -OC(O)OR), alkoxy groups (e.g., -OR), acetals (e.g., -C(OR)2R', where each OR is an alkoxy group which may be the same or different, and R' is an alkyl or alkenyl group), phosphates (e.g., P(O)₄³⁻), thiols (e.g., -SH), sulfoxides (e.g., -S(O)R), sulfinic acids (e.g., -S(O)OH), sulfonic acids (e.g., -S(O)₂OH), thioaldehydes (e.g., -C(S)H), sulfates (e.g., S(O)₄²⁻), sulfonyl groups (e.g., -S(O)₂₋), amides (e.g., -C(O)NR₂ or -N(R)C(O)R), azido groups (e.g., -N₃), nitro groups (e.g., -NO₂), cyano groups (e.g., -CN), isocyano groups (e.g., -NC), acyloxy groups (e.g., -OC(O)R), amino groups (e.g., -NR₂, -NRH or -NH₂), carbamoyl groups (e.g., -OC(O)NR₂, -OC(O)NRH or - OC(O)NH₂), sulfonamides (e.g., -S(O)₂NR₂, -S(O)₂NRH, -S(O)₂NH₂, -N(R)S(O)₂R, - N(H)S(O)₂R, -N(R)S(O)₂H or -N(H)S(O)₂H), alkyl groups, alkenyl groups and cyclic groups (e.g., carbocyclic groups or heterocyclic groups). In any one of the foregoing, R is an alkyl or alkenyl group as defined herein.

In some embodiments, the substituents themselves may be further substituted with 1, 2, 3, 4, 5 or 6 substituents as defined herein. For example, a C₁₋₆ alkyl group may be further substituted with 1, 2, 3, 4, 5 or 6 substituents as described herein.

As used herein, the terms "about" and "approximately", when applied to one or more values of interest, refer to values that are similar to the specified reference value(s). In certain embodiments, the term "about" or "approximately" means a value falling in 25%, 20%, 19%, 18%, 17%, 16%, 15%, 14%, 13%, 12%, 11%, 10%, 9%, 8%, 7%, 6%, 5%, 4%, 3%, 2%, 1% or less in either direction (greater than or less than) the stated reference value, unless otherwise specified or evident from the context (except where such number would exceed 100% of a possible value). For example, when used in the context of the amount of a given compound in the lipid component of a nanoparticle composition, "about" can mean +/- 10% of the stated value. For example, a nanoparticle composition comprising a lipid component with about 40% of a given compound may comprise 30-50% of the compound.

As used herein, the term "compound" is intended to encompass all isomers and isotopes of the structures depicted. "Isotope" refers to atoms with the same atomic number but different mass numbers due to varying numbers of neutrons in their atomic nuclei. For instance, isotopes of hydrogen include tritium and deuterium. Furthermore, the compounds, salts, or complexes disclosed herein can be prepared by combining them with solvents or water molecules through conventional methods to form solvates and hydrates.

As used herein, the term "contact" means establishing a physical connection between two or more entities. For example, contacting a mammalian cell with a nanoparticle composition means physically connecting the mammalian cell and the nanoparticle. Methods for contacting cells with external entities in vivo and in vitro are well known in the biological field. For example, a nanoparticle composition can be brought into contact with mammalian cells through different routes of administration (e.g., intravenous, intramuscular, intradermal, and subcutaneous), and may involve varying amounts of the nanoparticle composition. Furthermore, the nanoparticle composition may contact more than one type of mammalian cell.

As used herein, the term "delivery" means providing an entity to a destination. For example, delivering a therapeutic agent and/or prophylactic agent to a subject may involve administering to the subject a nanoparticle composition comprising the therapeutic agent and/or prophylactic agent (e.g., via intravenous, intramuscular, intradermal or subcutaneous routes). Administering a nanoparticle composition to a mammal or mammalian cells may involve contacting one or more cells with the nanoparticle composition. "Local delivery" refers to delivering a drug primarily to a partial site of a subject, such as a certain organ, a certain tissue (e.g., a certain muscle), or the interior of a certain cell (e.g., the organ to which the drug is administered, the muscle to which the drug is administered, the cell to which the drug is administered), with no or substantially no delivery to organs, tissues, or cells other than the organs, tissues, or cells. For example, when a drug is administered into the kidney, the drug is locally delivered to other parts of the kidney, but not to organs, tissues, or cells outside the kidney (e.g., via the circulatory system). When a drug is administered into a certain muscle (e.g., the gastrocnemius muscle), the drug is locally delivered to other parts of the muscle, but not to organs, tissues, or cells outside the muscle (e.g., via the circulatory system).

In certain embodiments, nanoparticle compositions comprising compounds according to formula (I) exhibit essentially the same level of local delivery effect, regardless of the route of administration. For instance, when certain compounds disclosed herein are used for intravenous or intramuscular delivery of therapeutic and/or prophylactic agents, they exhibit similar local delivery. In some embodiments, intramuscular injection is superior to intravenous injection.

As used herein, the terms "specific delivery" and "local delivery" refer to the delivery of a greater amount (e.g., at least 1.5-fold, at least 2-fold, at least 3-fold, at least 4-fold, at least 5-fold, at least 6-fold, at least 7-fold, at least 8-fold, at least 9-fold, at least 10-fold or more) of a therapeutic agent and/or prophylactic agent via nanoparticles to a target cell, tissue or organ of interest (e.g., mammalian muscle) compared to non-target tissues (e.g., mammalian liver). The level of nanoparticle delivery to a specific tissue can be determined by the following comparisons: comparing the amount of protein produced in the tissue to the weight of the tissue, comparing the amount of therapeutic and/or prophylactic agent in the tissue to the weight of the tissue, comparing the amount of protein produced in the tissue to the total amount of protein in the tissue, or comparing the amount of therapeutic and/or prophylactic agent in the tissue to the total amount of therapeutic and/or prophylactic agent in said tissue. It should be understood that the ability of nanoparticles to specifically deliver to target tissues need not be determined in the subject receiving treatment; it can be determined in a surrogate such as an animal model (e.g., a rat model).

As used herein, "encapsulation efficiency" refers to the amount of the therapeutic agent and/or prophylactic agent that becomes part of the nanoparticle composition, relative to the initial total amount of the therapeutic agent and/or prophylactic agent used to prepare the nanoparticle composition. For example, if 97 mg of the therapeutic agent and/or prophylactic agent out of the total 100 mg initially provided for the composition is encapsulated in the nanoparticle composition, the encapsulation efficiency can be 97%.

As used herein, "expression" of a nucleic acid sequence comprises translation of mRNA into a polypeptide or protein and/or post-translational modification of the polypeptide or protein.

As used herein, the term "in vitro" refers to an event that occurs in an artificial environment, e.g., in a test tube or reaction vessel, in a cell culture, in a Petri dish, etc., rather than in a living organism (e.g., an animal, plant or microorganism). As used herein, the term "in vivo" refers to an event that occurs in a living organism (e.g., an animal, plant or microorganism, or a cell or tissue thereof). As used herein, the term "ex vivo" refers to an event that occurs outside a living organism (e.g., an animal, plant or microorganism, or a cell or tissue thereof). An ex vivo event may occur in an environment with minimal changes compared with the natural (e.g., in vivo) environment.

As used herein, the term "isomer" refers to any geometric isomer, tautomer, zwitterion, stereoisomer, enantiomer or diastereomer of a compound. A compound may contain one or more chiral centers and/or double bonds, and thus may exist as stereoisomers. This disclosure encompasses any and all isomers of the compounds described herein, including stereoisomerically pure forms as well as mixtures of enantiomers and stereoisomers, e.g., racemates. Mixtures of enantiomeric and stereoisomeric compounds, as well as methods for resolving them into their enantiomeric or stereoisomeric components, are well known in the art.

As used herein, the term "lipid component" refers to a component of a nanoparticle composition comprising one or more lipids. For example, the lipid component may comprise one or more cationic/ionizable, pegylated, structural or other lipids, such as phospholipids.

As used herein, "administration" may comprise intravenous, intramuscular, intradermal, subcutaneous, or other methods of delivering the composition to a subject. The administration method can be selected to achieve targeted delivery (e.g., specific delivery) to a particular area or system of the body.

As used herein, "modified" means non-natural. For example, RNA may be a modified RNA. That is, RNA may comprise one or more non-naturally occurring nucleobases, nucleosides, nucleotides or linkers. RNA further comprises any sequence optimization performed on the RNA.

As used herein, a "nanoparticle composition" refers to a composition comprising one or more lipids. The size of a nanoparticle composition is typically on the micrometer scale or smaller, and it may comprise a lipid bilayer. Nanoparticle compositions comprise lipid nanoparticles (LNP), liposomes (e.g., lipid vesicles) and lipoplexes. For example, a nanoparticle composition may be a liposome with a lipid bilayer having a diameter of 500 nm or less.

As used herein, a "patient" refers to a subject who may seek or require treatment, require treatment, is undergoing treatment, will receive treatment, or is under the care of a trained professional for a specific disease or disorder.

As used herein, a "PEG lipid" or " PEGylated lipid " refers to a lipid comprising a polyethylene glycol moiety.

As used herein, the phrase "pharmaceutically acceptable" refers to those compounds, materials, compositions and/or dosage forms which, in the scope of sound medical judgment, are suitable for contact with human and animal tissues without excessive toxicity, irritation, allergic response or other problems or complications, and are commensurate with a reasonable benefit/risk ratio.

As used herein, the phrase "pharmaceutically acceptable excipients" refers to any ingredients other than the compounds described herein (e.g., carriers capable of suspending, complexing or dissolving the active compounds) and which are substantially non-toxic and innocuous in nature. Excipients may include, for example: antiadherents, antioxidants, binders, coating agents, compression aids, disintegrants, dyes (colorants), emollients, emulsifiers, fillers (diluents), film-forming agents or coating agents, fragrances, flavors, glidants (flow enhancers), lubricants, preservatives, printing inks, adsorbents, suspending agents or dispersing agents, sweeteners, and water of hydration. Exemplary excipients include, but are not limited to: butylated hydroxytoluene (BHT), calcium carbonate, calcium phosphate (dibasic), calcium stearate, croscarmellose sodium, crospovidone, citric acid, povidone, cysteine, ethyl cellulose, gelatin, hydroxypropyl cellulose, hydroxypropyl methylcellulose, lactose, magnesium stearate, maltitol, mannitol, methionine, methyl cellulose, methylparaben, microcrystalline cellulose, polyethylene glycol, polyvinylpyrrolidone, povidone, pregelatinized starch, propylparaben, retinyl palmitate, shellac, silicon dioxide, sodium carboxymethylcellulose, sodium citrate, sodium starch glycolate, sorbitol, starch (corn), stearic acid, sucrose, talc, titanium dioxide, vitamin A, vitamin E (α-tocopherol), vitamin C, xylitol, and other substances disclosed herein.

In this specification, the structural formulas of the compounds are used to represent certain isomers for convenience in some cases; however, this disclosure encompasses all isomers, such as geometric isomers, optical isomers based on asymmetric carbons, stereoisomers, tautomers and the like. It should be understood that not all isomers may exhibit the same level of activity.

For compounds represented by the chemical formulae herein, crystal polymorphism may exist. It should be noted that any crystalline form, mixture of crystalline forms, or their anhydrides or hydrates are included in the scope of this disclosure. The terms "crystalline polymorph," "polymorph," or "crystal form" refer to crystalline structures in which a compound (or its salt or solvate) can crystallize in different crystal packing arrangements, all having the same elemental composition. Different polymorphs typically exhibit different X-ray diffraction patterns, infrared spectra, melting points, densities, hardness, crystal shapes, optical and electrical properties, stability, and solubility. Recrystallization solvents, crystallization rate, storage temperature, and other factors can lead to one polymorph predominating. Crystalline polymorphs of a compound can be prepared by crystallization under different conditions.

The composition further comprises a salt of one or more compounds. The salt may be a pharmaceutically acceptable salt. As used herein, "pharmaceutically acceptable salt" refers to a derivative of the disclosed compound wherein the parent compound is modified by converting an existing acid or base moiety into its salt form (e.g., by reacting a free base with a suitable organic acid). Examples of pharmaceutically acceptable salts include, but are not limited to, inorganic or organic acid salts of basic residues such as amines; alkali metal or organic salts of acidic residues such as carboxylic acids, and the like.

Representative acid addition salts include acetate, adipate, alginate, ascorbate, aspartate, besylate, benzoate, bisulfate, borate, butyrate, camphorate, camsylate, citrate, cyclopentanepropionate, digluconate, dodecyl sulfate, edisylate, fumarate, glucoheptonate, glycerophosphate, hemisulfate, heptanoate, hexanoate, hydrobromide, hydrochloride, hydroiodide, 2-isethionate, lactobionate, lactate, laurate, dodecyl sulfate, malate, maleate, malonate, mesylate, 2-napsylate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, tosylate, undecanoate, valerate and the like.

Representative alkali metal or alkaline earth metal salts include sodium, lithium, potassium, calcium, magnesium and the like, as well as non-toxic ammonium, quaternary ammonium and amine cations, including but not limited to ammonium, tetramethylammonium, tetraethylammonium, methylamine, dimethylamine, trimethylamine, triethylamine, ethylamine and the like.

The pharmaceutically acceptable salts disclosed herein include, for example, conventional non-toxic salts of the parent compound formed from non-toxic inorganic acids or organic acids.

The pharmaceutically acceptable salts disclosed herein can be synthesized from the parent compounds comprising basic or acidic moieties by conventional chemical methods. Generally, such salts are prepared by reacting the free acid or base forms of these compounds with a stoichiometric amount of the appropriate base or acid in water, an organic solvent, or a mixture of both; generally, a non-aqueous medium such as diethyl ether, ethyl acetate, ethanol, isopropanol, or acetonitrile is preferred. A list of suitable salts can be found in any edition of Remington's Pharmaceutical Sciences, which is incorporated herein by reference in its entirety.

As used herein, "phospholipid" refers to a lipid comprising a phosphate moiety and one or more carbon chains, such as unsaturated fatty acid chains. Phospholipids may comprise one or more multiple bonds (e.g., double or triple bonds) (e.g., one or more degrees of unsaturation). Certain phospholipids can promote fusion with membranes. For example, cationic phospholipids can interact with one or more negatively charged phospholipids of a membrane (e.g., a cell membrane or intracellular membrane). The fusion of phospholipids with a membrane can allow one or more components of a lipid-containing composition to pass through the membrane, thereby allowing, for example, the delivery of one or more components to a cell.

As used herein, the term "polypeptide" or "polypeptide of interest" refers to a polymer of amino acid residues typically linked by peptide bonds, which may be naturally occurring (e.g., isolated or purified) or synthetically produced.

As used herein, "RNA" refers to ribonucleic acid that may be naturally or non-naturally occurring. For example, RNA may comprise modified and/or non-naturally occurring components, such as one or more nucleobases, nucleosides, nucleotides, or linkers. RNA may comprise cap structures, chain-terminating nucleotides, stem-loops, polyA sequences, and/or polyadenylation signals. RNA may have a nucleotide sequence that encodes a polypeptide of interest. For example, RNA may be messenger RNA (mRNA). Translation of mRNA encoding a specific polypeptide, such as in vivo translation of mRNA in mammalian cells, can produce the encoded polypeptide. RNA may be selected from small interfering RNA (siRNA), asymmetric interfering RNA (aiRNA), microRNA (miRNA), Dicer-substrate RNA (dsRNA), small hairpin RNA (shRNA), and mRNA.

As used herein, a "single unit dose" is the dose of any therapeutic agent administered in a single dose/single administration/single route/single point of contact, i.e., a single dosing event. As used herein, a "divided dose" is a single unit dose or total daily dose divided into two or more doses. As used herein, the "total daily dose" is the amount given or prescribed in a 24-hour period. It may be administered as a single unit dose.

As used herein, the term "size" or "average size" in the context of nanoparticle compositions refers to the average diameter of the nanoparticles in the composition.

As used herein, the terms "subject," "individual," or "patient" refer to any organism to which the compositions according to this disclosure may be administered, for example, for experimental, diagnostic, prophylactic, and/or therapeutic purposes. Typical subjects include animals (e.g., mammals such as mice, rats, rabbits, non-human primates, and humans) and/or plants. Preferably, the subjects described herein suffer from a disease that would benefit from local drug delivery, such as a lesion in an organ or tissue where it is desirable to deliver the drug only to that organ or tissue.

As used herein, "target cell" refers to any one or more cells of interest. The cells may be found in vitro, in vivo, in situ, or in the tissues or organs of an organism. The organism may be an animal, preferably a mammal, more preferably a human, and most preferably a patient.

As used herein, "target tissue" refers to any one or more tissue types of interest in which delivery of a therapeutic and/or prophylactic agent will result in a desired biological and/or pharmacological effect. Examples of target tissues of interest include specific tissues, organs, and systems or combinations thereof. In specific applications, the target tissue may be the kidney, lung, spleen, vascular endothelium in blood vessels (e.g., in the coronary arteries or femoral artery), or tumor tissue (e.g., via intratumoral injection) or muscle (e.g., via intramuscular injection).

As used herein, the term "muscle" includes but is not limited to: back muscles (including trapezius, latissimus dorsi, levator scapulae, rhomboid muscles, erector spinae, splenius muscles, transversospinales, interspinales, intertransversarii); chest muscles (including pectoralis major, pectoralis minor, serratus anterior, external intercostal muscles, internal intercostal muscles, transversus thoracis); diaphragm; abdominal muscles (including rectus abdominis, external oblique, internal oblique, transversus abdominis, quadratus lumborum); perineal muscles; shoulder girdle muscles (including deltoid, supraspinatus, infraspinatus, teres minor, subscapularis, teres major); upper arm muscles (including biceps brachii, coracobrachialis, brachialis, triceps brachii, anconeus); forearm muscles (including brachioradialis, pronator teres, flexor carpi radialis, flexor carpi ulnaris, extensor carpi radialis longus, extensor carpi radialis brevis, extensor carpi ulnaris); hand muscles; pelvic girdle muscles (including iliopsoas, piriformis, gluteus maximus, gluteus medius, gluteus minimus); thigh muscles (including quadriceps femoris, sartorius, tensor fasciae latae, biceps femoris, semitendinosus, semimembranosus, pectineus, adductor longus, adductor brevis, adductor magnus, gracilis); calf muscles (including tibialis anterior, extensor digitorum longus, gastrocnemius, soleus, flexor digitorum longus, flexor hallucis longus, tibialis posterior, peroneus longus and peroneus brevis); foot muscles; platysma; sternocleidomastoid.

The term "therapeutic agent" or "preventive agent" refers to any agent that, when administered to a subject, has a therapeutic, diagnostic, and/or preventive effect and/or elicits a desired biological and/or pharmacological effect. Therapeutic agents are also referred to as "active substances" or "active agents," and include, but are not limited to, cytotoxins, radioisotopes, chemotherapeutic drugs, small molecule drugs, proteins, and nucleic acids, such as RNA.

As used herein, the term "therapeutically effective amount" refers to an amount that, when administered to a subject having or predisposed to an infection, disease, disorder, and/or condition, is effective to treat, ameliorate the symptoms of, diagnose, prevent, and/or delay the onset of the infection, disease, disorder, and/or condition.

As used herein, the term "transfection" refers to the introduction of a substance (e.g., RNA) into a cell. Transfection can occur, for example, in vitro, ex vivo, or in vivo.

As used herein, the term "treatment" refers to the partial or complete alleviation, amelioration, improvement, relief, or delay of the onset of a particular infection, disease, disorder, and/or condition, inhibiting its progression, reducing its severity, and/or reducing its incidence. For example, "treating" cancer may refer to inhibiting the survival, growth, and/or spread of a tumor. To reduce risk, treatment may be administered to subjects who do not exhibit signs of the disease, disorder, and/or condition, and/or to subjects who exhibit only early signs of the disease, disorder, and/or condition, to prevent the development of pathology associated with the disease, disorder, and/or condition.

This disclosure further encompasses nanoparticle compositions comprising a lipid component, wherein the lipid component comprises a compound of formula (I) as described herein.

Nanoparticle compositions comprise, for example, lipid nanoparticles (LNP), liposomes, lipid vesicles, and lipid complexes. In some embodiments, the nanoparticle composition is a vesicle comprising one or more lipid bilayers. In certain embodiments, the nanoparticle composition comprises two or more concentric bilayers separated by an aqueous compartment. The lipid bilayers may be functionalized and/or cross-linked to each other. The lipid bilayers may comprise one or more ligands, proteins, or channels.

The nanoparticle composition comprises a lipid component, which comprises at least one compound according to formula (I). For example, the lipid component of the nanoparticle composition may comprise one or more of compounds 1-10. The nanoparticle composition may further comprise various other components. For example, in addition to the lipid of formula (I), the nanoparticle composition may comprise one or more cationic lipids.

The lipid component of the nanoparticle composition may comprise one or more PEG or PEG-modified lipids. Such substances may alternatively be referred to as polyethyleneglycolated lipids. PEG lipids are lipids modified with polyethylene glycol. PEG lipids may be selected from PEG-modified phosphatidylethanolamine, PEG-modified phosphatidic acid, PEG-modified ceramide, PEG-modified dialkylamine, PEG-modified diacylglycerol, PEG-modified dialkylglycerol, and mixtures thereof. For example, the PEG lipid may be PEG-c-DOMG, PEG-DMG, PEG-DLPE, PEG-DMPE, PEG-DPPC, or PEG-DSPE lipid.

The lipid component of the nanoparticle composition may comprise one or more structural lipids. The structural lipids may be selected from, but are not limited to, cholesterol, coprostanol, sitosterol, ergosterol, campesterol, stigmasterol, brassicasterol, tomatine, tomatine, ursolic acid, α-tocopherol, and mixtures thereof. In some embodiments, the structural lipid is cholesterol. In some embodiments, the structural lipid comprises cholesterol and a corticosteroid (e.g., prednisolone, dexamethasone, prednisone and hydrocortisone) or a combination thereof.

The lipid component of the nanoparticle composition may comprise one or more phospholipids, e.g., one or more unsaturated lipids. The phospholipids can assemble into one or more lipid bilayers. Generally, a phospholipid may comprise a phospholipid moiety and one or more fatty acid moieties. For example, the phospholipid may be a lipid according to formula (II). 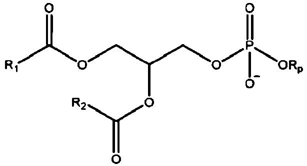 wherein Rp represents the phospholipid moiety, and R₁ and R₂ represent fatty acid moieties with or without unsaturation, which may be the same or different. The phospholipid moiety may be selected from phosphatidylcholine, phosphatidylethanolamine, phosphatidylglycerol, phosphatidylserine, phosphatidic acid, 2-lysophosphatidylcholine, and sphingomyelin. The fatty acid moiety may be selected from lauric acid, myristic acid, myristoleic acid, palmitic acid, palmitoleic acid, stearic acid, oleic acid, linoleic acid, α-linolenic acid, erucic acid, phytanic acid, arachidic acid, arachidonic acid, eicosapentaenoic acid, behenic acid, docosapentaenoic acid, and docosahexaenoic acid.

The phospholipids can be used in the compositions and methods described herein may be selected from DSPC, DOPE, DLPC, DMPC, DOPC, DPPC, DUPC, POPC, OChemsPC, DOPG and sphingomyelin. In some embodiments, the nanoparticle composition comprises DSPC. In some embodiments, the nanoparticle composition comprises DOPE. In some embodiments, the nanoparticle composition comprises DSPC and/or DOPE.

In some embodiments, the nanoparticle compositions described herein comprise a compound of formula (I), PEG lipid, DSPC, and cholesterol.

In some embodiments, the nanoparticle compositions comprising one or more lipids described herein may further comprise one or more adjuvants, such as aluminum hydroxide.

Therapeutic and/or preventive agents

The nanoparticle composition may comprise one or more therapeutic and/or prophylactic agents. The present disclosure provides methods for delivering therapeutic and/or prophylactic agents to mammalian cells or organs, producing polypeptides of interest in mammalian cells, and treating diseases or conditions in mammals in need thereof, comprising administering to and/or contacting mammalian cells with a nanoparticle composition comprising therapeutic and/or prophylactic agents.

A therapeutic and/or prophylactic agent can be a substance that, once delivered to a cell or organ, brings about a desired change in the cell, organ, or other body tissue or system. Such agents can be used to treat one or more diseases or conditions. In some embodiments, the therapeutic and/or prophylactic agent is a small molecule drug that can be used to treat a specific disease or condition.

Examples of drugs can be used in the nanoparticle composition include, but are not limited to, antineoplastic agents (e.g., doxorubicin, mitoxantrone, camptothecin, cisplatin, bleomycin, cyclophosphamide, streptozocin, dactinomycin, vincristine, vinblastine, cytosine arabinoside, anthracyclines, alkylating agents, platinum compounds, antimetabolites and nucleoside analogs such as methotrexate and purine and pyrimidine analogs), anti-infective agents, local anesthetics (e.g., dibucaine and chlorpromazine), beta-adrenergic blockers (e.g., propranolol, timolol and labetalol), antihypertensive agents (e.g., clonidine and hydralazine), antidepressants (e.g., imipramine, amitriptyline and doxepin), antiepileptics (e.g., phenytoin sodium), antihistamines (e.g., diphenhydramine, chlorpheniramine and promethazine), antibiotics/antibacterial agents (e.g., gentamicin, ciprofloxacin and cefoxitin), antifungal agents (e.g., miconazole, terconazole, econazole, isoconazole, butoconazole, clotrimazole, itraconazole, nystatin, naftifine and amphotericin B), antiparasitic agents, hormones, hormone antagonists, immunomodulators, neurotransmitter antagonists, antiglaucoma agents, vitamins, anesthetics and imaging agents.

In some embodiments, the therapeutic agent and/or prophylactic agent is a cytotoxin, radioactive ion, chemotherapeutic agent, vaccine, compound that elicits an immune response, and/or another therapeutic agent and/or prophylactic agent. A cytotoxin or cytotoxic agent includes any agent that may be detrimental to cells. Examples include, but are not limited to, paclitaxel, cytochalasin B, gramicidin D, ethidium bromide, emetine, mitomycin, etoposide, teniposide, vincristine, vinblastine, colchicine, doxorubicin, daunorubicin, dihydroxyanthracenedione, mitoxantrone, mithramycin, dactinomycin D, 1-dehydrotestosterone, glucocorticoids, procaine, tetracaine, lidocaine, propranolol, puromycin, maytansine, e.g., maytansinol, leinamycin, and analogs or homologs thereof. Radioactive ions include, but are not limited to, iodine (e.g., iodine-125 or iodine-131), strontium-89, phosphorus, palladium, cesium, iridium, phosphate, cobalt, yttrium-90, samarium-153, and praseodymium.

Other therapeutic and/or prophylactic agents include, but are not limited to, antimetabolites (e.g., methotrexate, 6-mercaptopurine, 6-thioguanine, cytarabine, 5-fluorouracil, dacarbazine), alkylating agents (e.g., chlormethine, thiotepa, chlorambucil, leinamycin, melphalan, carmustine (BSNU), lomustine (CCNU), cyclophosphamide, busulfan, dibromomannitol, streptozocin, mitomycin C, and cisdichlorodiammineplatinum(II) (DDP) cisplatin), anthracyclines (e.g., daunorubicin (formerly known as daunomycin) and doxorubicin), antibiotics (e.g., dactinomycin (formerly known as actinomycin), bleomycin, mithramycin, and anthramycin (AMC), and antimitotic agents (e.g., vincristine, vinblastine, paclitaxel, and maytansinoids).

In other embodiments, the therapeutic agent and/or prophylactic agent is a protein. Therapeutic proteins useful in the nanoparticles of the present disclosure include, but are not limited to, gentamicin, amikacin, insulin, erythropoietin (EPO), granulocyte colony-stimulating factor (G-CSF), granulocyte-macrophage colony-stimulating factor (GM-CSF), Factor VIR, luteinizing hormone-releasing hormone (LHRH) analogs, interferons, heparin, hepatitis B surface antigen, typhoid vaccines, and cholera vaccines.

In some embodiments, the therapeutic agent is a polynucleotide or nucleic acid (e.g., ribonucleic acid or deoxyribonucleic acid). The term "polynucleotide," in its broadest sense, includes any compound and/or substance that is or can be incorporated into an oligonucleotide chain. Exemplary polynucleotides used according to this disclosure include, but are not limited to, deoxyribonucleic acid (DNA), ribonucleic acid (RNA) including messenger RNA (mRNA), hybrids thereof, one or more RNAi inducers, RNAi agents, siRNA, shRNA, miRNA, antisense RNA, ribozymes, catalytic DNA, RNA that induces triple helix formation, aptamers, vectors, and the like.

In some embodiments, the therapeutic and/or prophylactic agent is RNA. The RNA suitable for use in the compositions and methods described herein may be selected from, but is not limited to, short-chain RNA, antagonists, antisense strands, ribozymes, small interfering RNA (siRNA), asymmetric interfering RNA (aiRNA), microRNA (miRNA), Dicer-substrate RNA, small hairpin RNA (shRNA), transfer RNA (tRNA), messenger RNA (mRNA), and mixtures thereof. In certain embodiments, the RNA is mRNA or siRNA.

In some embodiments, the therapeutic and/or prophylactic agent is mRNA. The mRNA can encode any polypeptide of interest, including any naturally or non-naturally occurring or otherwise modified polypeptide. The polypeptide encoded by the mRNA can be of any size and can have any secondary structure or activity. In some embodiments, the polypeptide encoded by the mRNA may have a therapeutic effect when expressed in a cell.

In other embodiments, the therapeutic and/or prophylactic agent is siRNA. The siRNA may be capable of selectively knocking down or downregulating the expression of a gene of interest. For example, the siRNA may be selected to silence a gene associated with a particular disease or condition when a nanoparticle composition comprising the siRNA is administered to a subject in need thereof. The siRNA may comprise a sequence complementary to the mRNA sequence encoding the gene or protein of interest. In some embodiments, the siRNA may be an immunomodulatory siRNA.

In some embodiments, the therapeutic and/or prophylactic agent is an shRNA or a vector or plasmid encoding it. The shRNA can be produced in the target cell after delivery of the appropriate construct to the cell nucleus. Constructs and mechanisms related to shRNA are well known in the relevant art.

Nucleic acids and polynucleotides used in the present disclosure generally comprise a first region of linked nucleosides encoding a polypeptide of interest (e.g., a coding region), a first flanking region located at the 5'-end of the first region (e.g., a 5'-UTR), a second flanking region located at the 3'-end of the first region (e.g., a 3'-UTR), at least one 5'-cap region, and a 3'-stabilizing region. In some embodiments, the nucleic acid or polynucleotide further comprises a poly-A region or a Kozak sequence (e.g., in the 5'-UTR). In some cases, the polynucleotide may comprise one or more intronic nucleotide sequences capable of being excised from the polynucleotide. In some embodiments, the polynucleotide or nucleic acid (e.g., mRNA) may comprise a 5' cap structure, a chain-terminating nucleotide, a stem-loop, a polyadenylation sequence, and/or a polyadenylation signal. Any region of the nucleic acid may comprise one or more alternative components (e.g., alternative nucleosides).

Generally, the shortest length of a polynucleotide can be the length of a polynucleotide sequence sufficient to encode a dipeptide. In another embodiment, the length of the polynucleotide sequence is sufficient to encode a tripeptide, tetrapeptide, pentapeptide, hexapeptide, heptapeptide, octapeptide, nonapeptide, or decapeptide. In some cases, the length of the polynucleotide is at least 30, at least 35, at least 40, at least 45, at least 50, at least 55, at least 60, at least 80, at least 90, at least 100, at least 120, at least 150, at least 180, at least 200, at least 300, at least 400, at least 500, at least 600, at least 700, at least 800, at least 900, at least 1000, at least 2000, at least 3000, at least 4000, or at least 5000 nucleotides.

Nucleic acids and polynucleotides may comprise one or more naturally occurring components, including any typical nucleotides A (adenosine), G (guanosine), C (cytidine), U (uridine), or T (thymidine). In one embodiment, all or substantially all of the nucleotides comprise (a) a 5'-UTR, (b) an open reading frame (ORF), (c) a 3'-UTR, (d) a polyadenylation tail, and any combination of (a), (b), (c), or (d) above.

Polynucleotides and nucleic acids can include one or more modified (e.g., altered or substituted) nucleobases, nucleosides, nucleotides, or combinations thereof. Nucleic acids and polynucleotides used in nanoparticle compositions may include any useful modifications or alterations, such as those to the nucleobases, sugars, or internucleoside linkages (e.g., the phosphodiester bond/phosphodiester backbone).

In some cases, the nucleic acid does not substantially induce an innate immune response in the cells into which the polynucleotide (e.g., mRNA) is introduced.

### Other components

In addition to those described in the preceding sections, the nanoparticle composition may comprise one or more components. For example, the nanoparticle composition may comprise one or more hydrophobic small molecules, such as vitamins (e.g., vitamin A or vitamin E) or sterols.

The nanoparticle composition may further comprise one or more penetration enhancer molecules, carbohydrates, polymers, surface-altering agents, or other components. The penetration enhancer molecules may be, for example, those described in U.S. Patent Application Publication No. 2005/0222064. Carbohydrates may include monosaccharides (e.g., glucose) and polysaccharides (e.g., glycogen and derivatives and analogs thereof). Polymers may be included in and/or used to encapsulate or partially encapsulate the nanoparticle composition. The polymers may be biodegradable and/or biocompatible. The polymers may be selected from, but are not limited to, polyamines, polyethers, polyamides, polyesters, polyurethanes, polyureas, polycarbonates, polystyrenes, polyimides, polysulfones, polyurethanes, polyacetylenes, polyethylenes, polyethyleneimines, polyisocyanates, polyacrylates, polymethacrylates, polyacrylonitriles, and polyarylates. Surface-altering agents may include, but are not limited to, anionic proteins (e.g., bovine serum albumin), surfactants (e.g., cationic surfactants, e.g., dimethyldioctadecylammonium bromide), sugars or sugar derivatives (e.g., cyclodextrins), nucleic acids, polymers (e.g., heparin, polyethylene glycol, and poloxamers), mucolytics (e.g., acetylcysteine, neltenexine, and erdosteine), and DNases (e.g., rhDNase). The surface-altering agents may be disposed in the nanoparticles and/or on the surface of the nanoparticle composition (e.g., via coating, adsorption, covalent attachment, or other processes).

The nanoparticle composition may also comprise one or more functionalized lipids. For example, the lipids can be functionalized with alkyne groups, which can undergo cycloaddition reactions when exposed to azides under appropriate reaction conditions. Specifically, the lipid bilayer can be functionalized in this way with one or more groups that can be used to facilitate membrane penetration, cell recognition, or imaging.

The surface of the nanoparticle composition can also be conjugated with one or more useful antibodies. Functional groups and conjugates for targeted cell delivery, imaging, and membrane penetration are well known in the art.

In addition to these components, the nanoparticle composition may comprise any substance useful in pharmaceutical compositions. For example, the nanoparticle composition may comprise one or more pharmaceutically acceptable excipients or auxiliary components, such as but not limited to one or more solvents, dispersion media, diluents, dispersion aids, suspension aids, granulation aids, disintegrants, fillers, glidants, liquid carriers, binders, surfactants, isotonic agents, thickeners or emulsifiers, buffers, lubricants, oils, preservatives, and other substances. Excipients such as waxes, butters, colorants, coating agents, flavoring agents, and fragrances may also be included. Pharmaceutically acceptable excipients are well known in the art (see, e.g., Remington's The Science and Practice of Pharmacy, 21st Edition).

The lipid component of the nanoparticle composition may comprise, for example, lipids according to formula (I), phospholipids (e.g., unsaturated lipids such as DOPE or DSPC), PEG lipids, and structural lipids. Each lipid component may be provided in a specific molar fraction. In certain embodiments, the lipid component of the nanoparticle composition comprises about 30 mol% to about 60 mol% of the compound of formula (I), about 0 mol% to about 30 mol% of phospholipids, about 18.5 mol% to about 48.5 mol% of structural lipids, and about 0 mol% to about 10 mol% of PEG lipids. In some embodiments, the lipid component of the nanoparticle composition comprises about 35 mol% to about 55 mol% of the compound of formula (I), about 5 mol% to about 25 mol% of phospholipids, about 30 mol% to about 40 mol% of structural lipids, and about 0 mol% to about 10 mol% of PEG lipids. In specific embodiments, the lipid component comprises about 50 mol% of the compound of formula (I), about 10 mol% of phospholipids, about 38.5 mol% of structural lipids, and about 1.5 mol% of PEG lipids. In another embodiment, the lipid component comprises about 50 mol% of the compound of formula (I), about 10 mol% of phospholipids, about 38.5 mol% of structural lipids, and about 3.05 mol% of PEG lipids. In another specific embodiment, the lipid component comprises about 40 mol% of the compound of formula (I), about 20 mol% of phospholipids, about 38.5 mol% of structural lipids, and about 1.5 mol% of PEG lipids. In some embodiments, the phospholipid may be DOPE or DSPC. In other embodiments, the PEG lipid may be PEG-DMG (e.g., PEG2000-DMG) and/or the structural lipid may be cholesterol.

Alternatively, the lipid component may be quantified in molar parts; for example, the lipid component of the nanoparticle composition comprises about 30 molar parts to about 60 molar parts of the compound of formula (I), about 0 molar parts to about 30 molar parts of phospholipids, about 18.5 molar parts to about 48.5 molar parts of structural lipids, and about 0 molar parts to about 10 molar parts of PEG lipids. In some embodiments, the lipid component of the nanoparticle composition comprises about 35 molar parts to about 55 molar parts of the compound of formula (I), about 5 molar parts to about 25 molar parts of phospholipids, about 30 molar parts to about 40 molar parts of structural lipids, and about 0 mol% to about 10 molar parts of PEG lipids. In some embodiments, the lipid component comprises about 50 molar parts of the compound of formula (I), about 10 molar parts of phospholipids, about 38.5 molar parts of structural lipids, and about 1.5 molar parts of PEG lipids. In another embodiment, the lipid component comprises about 50 molar parts of the compound of formula (I), about 10 molar parts of phospholipids, about 38.5 molar parts of structural lipids, and about 3.05 molar parts of PEG lipids.

The amount of the therapeutic agent and/or prophylactic agent in the nanoparticle composition may depend on the size, composition, desired target and/or application of the nanoparticle composition, or other properties, as well as the properties of the therapeutic agent and/or prophylactic agent. For example, the amount of RNA used in the nanoparticle composition may depend on the size, sequence and other characteristics of the RNA. The relative amounts of the therapeutic and/or prophylactic agent and other components (e.g., lipids) in the nanoparticle composition may also vary. In some embodiments, the weight/weight ratio of the lipid component to the therapeutic agent and/or prophylactic agent in the nanoparticle composition may be from about 5:1 to about 60:1, e.g., 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1, 40:1, 45:1, 50:1, 60:1. For example, the weight/weight ratio of the lipid component to the therapeutic agent and/or prophylactic agent may be from about 10:1 to about 40:1. In certain embodiments, the weight/weight ratio is about 20:1. The amount of the therapeutic agent and/or prophylactic agent in the nanoparticle composition may be measured, for example, using absorption spectroscopy (e.g., ultraviolet-visible spectroscopy).

The average size of the nanoparticle composition can be between 10 nanometers and 100 nanometers, as measured by methods well-known in the art. For example, the average size can be from about 40 nm to about 150 nm, such as about 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm. In some embodiments, the average size of the nm particle composition may be from about 50 nm to about 100 nm, about 50 nm to about 90 nm, about 50 nm to about 80 nm, about 50 nm to about 70 nm, from about 50 nm to about 60 nm, about 60 nm to about 100 nm, about 60 nm to about 90 nm, about 60 nm to about 80 nm, about 60 nm to about 70 nm, about 70 nm to about 100 nm, about 70 nm to about 90 nm, about 70 nm to about 80 nm, about 80 nm to about 100 nm, about 80 nm to about 90 nm, or about 90 nm to about 100 nm. In some embodiments, the average size of the nm particle composition may be from about 70 nm to about 100 nm. In a particular embodiment, the average size may be about 70 nm.

The nanoparticle composition can be relatively uniform. The polydispersity index can be used to indicate the uniformity of the nanoparticle composition, such as the particle size distribution of the nanoparticle composition. A small polydispersity index (e.g., less than 0.3) generally indicates a narrow particle size distribution. The nanoparticle composition may have a polydispersity index of about 0 to about 0.25, such as 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23, 0.24, or 0.25. In some embodiments, the polydispersity index of the nanoparticle composition may be about 0.05 to about 0.10.

The encapsulation efficiency of the therapeutic agent and/or prophylactic agent describes the amount of the therapeutic agent and/or prophylactic agent that is encapsulated or otherwise associated with the nanoparticle composition after preparation, relative to the initial amount provided. The encapsulation efficiency may be measured, for example, by comparing the amount of the therapeutic agent and/or prophylactic agent in the solution containing the nanoparticle composition before and after disrupting the nanoparticle composition with one or more organic solvents or detergents. Fluorescence may be used to measure the amount of free therapeutic agent and/or prophylactic agent (e.g., RNA) in the solution. For the nanoparticle compositions described herein, the encapsulation efficiency of the therapeutic agent and/or prophylactic agent may be at least 50%, e.g., 50%, 55%, 60%, 65%, 70%, 75%, 80%, 85%, 90%, 91%, 92%, 93%, 94%, 95%, 96%, 97%, 98%, 99%, or 100%. In some embodiments, the encapsulation efficiency may be at least 80%. In certain embodiments, the encapsulation efficiency may be at least 90%.

The nanoparticle composition may optionally comprise one or more coatings. For example, the nanoparticle composition may be formulated into capsules, films, or tablets with coatings. The capsules, films, or tablets of the compositions described herein may have any useful size, tensile strength, hardness, or density.

The nanoparticle compositions of the present disclosure may be formulated into a variety of forms suitable for various administration routes, such as liquid dosage forms (e.g., emulsions, microemulsions, nanoemulsions, solutions, suspensions, syrups, and elixirs), injectable dosage forms, solid dosage forms (e.g., capsules, tablets, pills, powders, and granules), dosage forms for topical (including buccal and sublingual), transdermal and/or percutaneous administration (e.g., creams, ointments, pastes, lotions, gels, powders, solutions, sprays, inhalants, and patches), dosage forms for vaginal administration (e.g., vaginal suppositories, tampons, creams, gels, pastes, foams, and sprays), and dosage forms for implant administration (e.g., solids, semisolids, gels), suspensions, powders, and other dosage forms.

### Pharmaceutical composition

The nanoparticle composition can be formulated, in whole or in part, into a pharmaceutical composition. The pharmaceutical composition may comprise one or more nanoparticle compositions.

For example, a pharmaceutical composition may comprise one or more nanoparticle compositions, comprising one or more different therapeutic and/or prophylactic agents. The pharmaceutical composition may further comprise one or more pharmaceutically acceptable excipients, such as those described herein. General guidelines for formulating and manufacturing pharmaceutical compositions and agents can be found in Remington's The Science and Practice of Pharmacy, 21st Edition. Conventional excipients and auxiliary components may be used in any pharmaceutical composition, unless any conventional excipient or auxiliary component is incompatible with one or more components of the nanoparticle composition. The amount of excipients in the pharmaceutical composition can be determined as needed by those skilled in the art.

The relative amounts of one or more nanoparticle compositions, one or more pharmaceutically acceptable excipients, and/or any additional components in the disclosed pharmaceutical compositions will vary depending on the characteristics and size of the nanoparticles and/or the condition of the subject being treated, and further depending on the route of administration of the composition. For example, the pharmaceutical composition may comprise 0.1% to 100% (wt/wt) of one or more nanoparticle compositions.

In some embodiments, the nanoparticle compositions and/or pharmaceutical compositions of this disclosure are refrigerated or frozen for storage and/or transport (e.g., stored at a temperature of 4°C or lower, such as between approximately -150°C and approximately 0°C or between approximately -80°C and approximately -20°C (e.g., approximately -5°C, -10°C, -15°C, -20°C, -25°C, -30°C, -40°C, -50°C, -60°C, -70°C, -80°C, -90°C, -130°C, or -150°C).

The nanoparticle compositions and/or pharmaceutical compositions disclosed herein may be stable for at least 1 week, at least 2 weeks, at least 3 weeks, at least 4 weeks, at least 5 weeks, at least 6 weeks, at least 2 months, at least 4 months, at least 6 months, at least 8 months, at least 10 months, at least 12 months, at least 14 months, at least 16 months, at least 18 months, at least 20 months, at least 22 months, or at least 24 months, for example at a temperature of 4°C or lower (e.g., between about 4°C and -20°C). In one embodiment, the formulation is stable at 4°C for at least 4 weeks. In some embodiments, the pharmaceutical composition of the present disclosure comprises the nanoparticle composition disclosed herein and a pharmaceutically acceptable excipient selected from one or more of Tris, acetate (e.g., sodium acetate), citrate (e.g., sodium citrate), saline, PBS, and sucrose. In some embodiments, the pharmaceutical composition of the present disclosure has a pH between about 7 and 8 (e.g., 6.8, 6.9, 7.0, 7.1, 7.2, 7.3, 7.4, 7.5, 7.6, 7.7, 7.8, 7.9, or 8.0, or between 7.5 and 8 or between 7 and 7.8).

In the context of this disclosure, "stability" and "stable" refer to the resistance of the disclosed nanoparticle compositions and/or pharmaceutical compositions to chemical or physical changes (e.g., degradation, particle size changes, aggregation, changes in encapsulation, etc.) under given manufacturing, preparation, transportation, storage, and/or use conditions, such as when stresses like shear forces or freeze/thaw stresses are applied.

Nanoparticle compositions and/or pharmaceutical compositions comprising one or more nanoparticle compositions may be administered to any patient or subject, including those patients or subjects whose cells, tissues, organs, or systems, or combinations thereof, may benefit from the therapeutic effects provided by delivering one or more specific therapeutic and/or prophylactic agents.

Although the term "composition" primarily refers to compositions suitable for administration to humans, those skilled in the art will understand that such compositions are generally suitable for administration to any other mammal.

It is well known that compositions suitable for administration to humans can be modified to make them suitable for administration to various animals, and a skilled veterinary pharmacologist can design and/or carry out such modifications with only routine (if any) experimentation. The subjects to which the compositions are intended to be administered include, but are not limited to, humans, other primates, and other mammals, including commercially relevant mammals such as cattle, pigs, horses, sheep, cats, dogs, mice, and/or rats.

A pharmaceutical composition comprising one or more nanoparticle compositions can be prepared by any method known or later developed in the pharmacological field. Generally, such preparation methods involve combining the active ingredient with excipients and/or one or more other auxiliary components, and then, if desired or necessary, dividing, shaping, and/or packaging the product into the desired single or multiple forms. Multi dose units.

The pharmaceutical compositions disclosed herein may be prepared, packaged, and/or sold in bulk, as single unit doses, and/or as multiple single unit doses. As used herein, a "unit dose" is a discrete quantity of a pharmaceutical composition comprising a predetermined amount of active ingredient (e.g., a nanoparticle composition). The amount of active ingredient is generally equivalent to the dose of active ingredient to be administered to a subject and/or a convenient fraction of that dose, such as one-half or one-third of the dose.

The pharmaceutical composition may be formulated into a variety of forms suitable for multiple administration routes and methods. For example, the pharmaceutical composition may be formulated into liquid dosage forms (e.g., emulsions, microemulsions, nanoemulsions, solutions, suspensions, syrups, and elixirs), injectable dosage forms, solid dosage forms (e.g., capsules, tablets, pills, powders, and granules), dosage forms for topical (including buccal and sublingual), transdermal and/or percutaneous administration (e.g., creams, ointments, pastes, lotions, gels, powders, solutions, sprays, inhalants, and patches), dosage forms for vaginal administration (e.g., vaginal suppositories, tampons, creams, gels, pastes, foams, and sprays), dosage forms for implant administration (e.g., solids, semisolids, gels), suspensions, powders, and other dosage forms.

Liquid dosage forms for oral and parenteral administration include, but are not limited to, pharmaceutically acceptable emulsions, microemulsions, nanoemulsions, solutions, suspensions, syrups and/or elixirs. In addition to the active ingredients, the liquid dosage forms may comprise inert diluents commonly used in the art, such as water or other solvents, solubilizers and emulsifiers, e.g., ethanol, isopropanol, ethyl carbonate, ethyl acetate, benzyl alcohol, benzyl benzoate, propylene glycol, 1,3-butanediol, dimethylformamide, oils (in particular cottonseed oil, peanut oil, corn oil, germ oil, olive oil, castor oil and sesame oil), glycerol, tetrahydrofurfuryl alcohol, polyethylene glycols and sorbitan fatty acid esters, and mixtures thereof. Besides inert diluents, the oral compositions may comprise additional therapeutic and/or prophylactic agents, as well as supplementary agents such as wetting agents, emulsifiers and suspending agents, sweeteners, flavoring agents and/or fragrances. In certain embodiments for parenteral administration, the composition is mixed with solubilizers, e.g., alcohols, oils, modified oils, glycols, polysorbates, cyclodextrins, polymers and/or combinations thereof.

Injectable preparations, such as sterile injectable aqueous or oily suspensions, may be prepared using suitable dispersing, wetting, and/or suspending agents according to known techniques. Sterile injectable preparations may be sterile injectable solutions, suspensions, and/or emulsions in non-toxic parenterally acceptable diluents and/or solvents. Acceptable carriers and solvents that can be used comprise water, Ringer's solution, and isotonic sodium chloride solution. Sterile oils can be used as solvents or suspending media. For this purpose, any mild, non-volatile oil can be used, including synthetic monoglycerides or diglycerides. Fatty acids such as oleic acid can be used in the preparation of injectable formulations. Injectable preparations can be sterilized, for example, by filtration through a bacterial retention filter, and/or by incorporating a sterilizing agent in the form of a sterile solid composition, which can be dissolved or dispersed in sterile water or other sterile injectable medium before use.

Compositions for rectal or vaginal administration are usually suppositories, which can be prepared by mixing the composition with suitable non-irritating excipients that are solid at ambient temperature but liquid at body temperature, and thus melt in the rectum or vaginal cavity to release the active ingredients.

The present disclosure also contemplates the use of transdermal patches, which dosage forms can be prepared, for example, by dissolving and/or dispersing the compound in a suitable medium. Alternatively or additionally, the rate can be controlled by providing a ratecontrolling membrane and/or by dispersing the compound in a polymeric matrix and/or gel.

Suitable devices for delivering the intradermal drug compositions described herein include any type of liquid or solid injection device, such as conventional syringes, fine-gauge syringes, short-needle syringes, liquid jet injectors, compressed gas-accelerated powder injectors, ballistic powder delivery devices, etc.

The pharmaceutical compositions may be prepared, packaged, and/or sold in formulations suitable for administration via the buccal cavity or lungs. Such formulations may comprise dry particles comprising the active ingredient. Such compositions are conveniently in the form of dry powder for administration using devices containing a dry powder reservoir and/or using self-propelling solvent/powder dispensing containers. Such formulations can also be prepared, packaged, and/or sold as aqueous solutions and/or dilute alcoholic solutions and/or suspensions (optionally sterile, comprising the active ingredient), and can be conveniently administered using any nebulizing and/or atomizing device. Such formulations may further comprise one or more additional ingredients, including but not limited to flavoring agents such as sodium saccharin, volatile oils, buffering agents, surfactants, and/or preservatives such as methylparaben.

The formulations described herein that are suitable for pulmonary delivery may also be used for intranasal delivery of pharmaceutical compositions. Such formulations are administered via nasal inhalation, i.e., rapid inhalation through the nasal passages from a powder container held close to the nose. The pharmaceutical compositions may be prepared, packaged and/or sold in the form of preparations suitable for oral administration. Such preparations may be in the form of tablets and/or lozenges produced by conventional methods, for example.

### Methods and Uses

The present disclosure provides a method for producing a target polypeptide in mammalian cells. The method for polypeptide production involves contacting the cells with a nanoparticle composition comprising mRNA encoding the target polypeptide. When the cells are contacted with the nanoparticle composition, the mRNA can be internalized and translated in the cells to produce the target polypeptide.

Generally, the step of contacting mammalian cells with a nanoparticle composition containing mRNA encoding the target polypeptide can be performed in vivo, ex vivo, in culture, or in vitro. The amount of the nanoparticle composition and/or the amount of mRNA contacted with the cells may depend on the type of cells or tissue being contacted, the mode of administration, the physicochemical characteristics of the nanoparticle composition and mRNA (e.g., size, charge, and chemical composition), and other factors. Efficiency indicators may include polypeptide translation (indicated by polypeptide expression), mRNA degradation levels, and immune response indicators.

The step of contacting the nanoparticle composition containing mRNA with cells may involve or induce transfection. The phospholipids included in the lipid component of the nanoparticle composition can facilitate transfection and/or improve transfection efficiency, for example, by interacting with and/or fusing with the cell membrane or intracellular membranes. Transfection allows the mRNA to be translated in the cell.

In some embodiments, the nanoparticle compositions described herein can be used for treatment. For example, the mRNA contained in the nanoparticle composition can encode a therapeutic polypeptide (e.g., in the translatable region) and produce the therapeutic polypeptide after contact with and/or entry into (e.g., transfection of) cells. In other embodiments, the mRNA contained in the nanoparticle composition can encode a polypeptide that improves or increases the immunity of a subject.

In some embodiments, the mRNA included in the nanoparticle composition encodes a recombinant polypeptide that can replace one or more polypeptides that are substantially absent in the cells contacted by the nanoparticle composition. Alternatively, the recombinant polypeptide produced by mRNA translation can antagonize the activity of endogenous proteins in the cell, on the cell surface, or secreted by the cell. In another alternative, recombinant polypeptides produced through mRNA translation can indirectly or directly antagonize the activity of biological components in the cell, on the cell surface, or secreted from the cell. These antagonized biological components may include, but are not limited to, lipids (e.g., cholesterol), lipoproteins (e.g., low-density lipoprotein), nucleic acids, carbohydrates, and small molecule toxins.

### Methods for delivering therapeutic agents to cells and organs.

The present disclosure provides a method for delivering therapeutic and/or prophylactic agents to mammalian cells or organs. Delivering the therapeutic and/or prophylactic agents to cells involves administering to a subject a nanoparticle composition comprising the therapeutic and/or prophylactic agents, wherein administration of the composition involves contacting the cells with the composition. For example, proteins, cytotoxic agents, radioactive ions, chemotherapeutic agents or nucleic acids (e.g., RNA, such as mRNA or siRNA) can be delivered to cells or organs. In cases where the therapeutic and/or prophylactic agent is mRNA, when cells are contacted with the nanoparticle composition, the translatable mRNA can be translated in the cells to produce the polypeptide of interest. However, substantially non-translatable mRNA can also be delivered to cells.

In some embodiments, the nanoparticle composition can target specific cell types (e.g., cells of a specific organ or its system). For example, the nanoparticle composition comprising the therapeutic and/or prophylactic agent of interest can be specifically delivered to heart, liver, kidney, spleen, femur or lung of a mammal while being substantially not delivered to other cell types. Specific delivery to a specific cell type, organ, or its system or tissue means that a higher proportion of the nanoparticle composition comprising the therapeutic and/or prophylactic agent is delivered to the destination of interest (e.g., tissue) relative to other destinations. In certain embodiments, the tissue of interest is selected from the group consisting of heart, myocardium, liver, kidney, lung, spleen, femur, muscle, and tumor tissue (e.g., via intratumoral injection).

As another example of targeted or specific delivery, mRNA encoding a protein binding ligand (e.g., an antibody or its functional fragment, a scaffold protein, or a peptide) or receptor on the cell surface can be included in the nanoparticle composition. In some embodiments, the ligand may be a surface-bound antibody, which can allow for modulation of cell targeting specificity. These methods can improve the affinity and specificity of the targeting interaction. Ligands can be selected, for example, by technicians in the biological field based on the desired localization or function of the cell. Target cells may include, but are not limited to, cardiomyocytes, hepatocytes, epithelial cells, hematopoietic cells, endothelial cells, lung cells, bone cells, stem cells, mesenchymal cells, nerve cells, adipocytes, vascular smooth muscle cells, skeletal muscle cells, beta cells, pituitary cells, synovial lining cells, ovarian cells, testicular cells, fibroblasts, B cells, T cells, reticulocytes, leukocytes, granulocytes, and tumor cells.

### Methods for Treating Diseases and Disorders

The nanoparticle compositions can be used for treating diseases or disorders. In particular, such compositions can be used to treat diseases or disorders characterized by the deficiency or abnormality of protein or polypeptide activity. For example, nanoparticle compositions comprising mRNA encoding the deficient or abnormal polypeptide can be administered or delivered to cells. Subsequent translation of the mRNA can produce the polypeptide, thereby alleviating or eliminating the problems caused by the activity deficiency or abnormal activity induced by the polypeptide. The described methods and compositions can be used for treating acute diseases or disorders, such as sepsis, stroke and myocardial infarction. The therapeutic and/or prophylactic agents contained in the nanoparticle compositions may also be capable of altering the transcription rate of a given species, thereby affecting gene expression. Diseases and/or disorders characterized by dysfunctional or abnormal protein or polypeptide activity, for which the compositions can be administered, include, but are not limited to, rare diseases, infectious diseases (serving as vaccines and therapeutics), cancer and proliferative diseases, genetic diseases (e.g., cystic fibrosis), autoimmune diseases, diabetes, neurodegenerative diseases, cardiovascular and renovascular diseases, as well as metabolic diseases.

The present disclosure provides methods involving the administration of nanoparticle compositions comprising one or more therapeutic and/or prophylactic agents, as well as pharmaceutical compositions comprising the same. The terms therapeutic and prophylactic are used interchangeably herein. A therapeutic composition, or an imaging, diagnostic or prophylactic composition thereof, may be administered to a subject in any reasonable amount and by any administration route effective for preventing, treating, diagnosing or imaging a disease, disorder and/or condition. The specific amount to be administered to a subject may vary depending on the species, age and general condition of the subject, the specific components, and the mode of administration. The compositions according to the present disclosure may be formulated in dosage unit form to facilitate administration and uniformity of dosage. It should be understood, however, that the specific dosage amount of the compositions of the present disclosure will be determined by the attending physician in the scope of sound medical judgment.

Nanoparticle compositions comprising one or more therapeutic and/or prophylactic agents may be administered by any route. In some embodiments, the compositions, including prophylactic, diagnostic or imaging compositions comprising one or more nanoparticle compositions described herein, are administered by one or more of a variety of routes, including oral, intravenous, intramuscular, intraarterial, intramedullary, intrathecal, subcutaneous, intraventricular, transdermal or intradermal, intradermal, intrarectal, intravaginal, intraperitoneal, intraocular, subretinal, intravitreal, mucosal, intranasal, buccal, enteral, intratumoral, sublingual, intranasal; via intratracheal instillation, bronchial instillation and/or inhalation, as oral sprays and/or powders, nasal sprays and/or aerosols, and/or via portal vein catheterization. Local administration is preferred, e.g., intramuscular, subcutaneous, transdermal or intradermal, intradermal, intraperitoneal, intraocular, subretinal, intravitreal, mucosal, intranasal, buccal, intratumoral, intranasal. The suitable route of administration will depend on various factors, including the nature of the nanoparticle composition, the therapeutic and/or prophylactic agent, the patient's condition, etc.

In certain embodiments, the compositions according to the present disclosure may be administered at a level sufficient to deliver the following doses: about 0.0001 mg/kg to about 10 mg/kg, about 0.001 mg/kg to about 10 mg/kg, about 0.005 mg/kg to about 10 mg/kg, about 0.01 mg/kg to about 10 mg/kg, about 0.05 mg/kg to about 10 mg/kg, about 0.1 mg/kg to about 10 mg/kg, about 1 mg/kg to about 10 mg/kg, about 2 mg/kg to about 10 mg/kg, about 5 mg/kg to about 10 mg/kg, about 0.0001 mg/kg to about 5 mg/kg, about 0.001 mg/kg to about 5 mg/kg, about 0.005 mg/kg to about 5 mg/kg, about 0.01 mg/kg to about 5 mg/kg, about 0.05 mg/kg to about 5 mg/kg, about 0.1 mg/kg to about 5 mg/kg, about 1 mg/kg to about 5 mg/kg, about 2 mg/kg to about 5 mg/kg, about 0.0001 mg/kg to about 2.5 mg/kg, about 0.001 mg/kg to about 2.5 mg/kg, about 0.005 mg/kg to about 2.5 mg/kg, about 0.01 mg/kg to about 2.5 mg/kg, about 0.05 mg/kg to about 2.5 mg/kg, about 0.1 mg/kg to about 2.5 mg/kg, about 1 mg/kg to about 2.5 mg/kg, about 2 mg/kg to about 2.5 mg/kg, about 0.0001 mg/kg to about 1 mg/kg, about 0.001 mg/kg to about 1 mg/kg, about 0.005 mg/kg to about 1 mg/kg, about 0.01 mg/kg to about 1 mg/kg, about 0.05 mg/kg to about 1 mg/kg, about 0.1 mg/kg to about 1 mg/kg, about 0.0001 mg/kg to about 0.25 mg/kg, about 0.001 mg/kg to about 0.25 mg/kg, about 0.005 mg/kg to about 0.25 mg/kg, about 0.01 mg/kg to about 0.25 mg/kg, about 0.05 mg/kg to about 0.25 mg/kg, or about 0.1 mg/kg to about 0.25 mg/kg of the therapeutic and/or prophylactic agent (e.g., mRNA).

The dose may be administered once or multiple times daily in the same or different amounts to achieve the desired level of mRNA expression and/or therapeutic, diagnostic, prophylactic, or imaging effect. The desired dose may be delivered, for example, three times a day, twice a day, once a day, every other day, every three days, weekly, every two weeks, every three weeks, or every four weeks. In some embodiments, multiple administrations (e.g., two, three, four, five, six, seven, eight, nine, ten, eleven, twelve, thirteen, fourteen, or more administrations) may be used to deliver the desired dose.

Nanoparticle compositions comprising one or more therapeutic and/or prophylactic agents may be used in combination with one or more other therapeutic, prophylactic, diagnostic, or imaging agents. Each agent will be administered at a dose and/or schedule determined for that agent. The therapeutic, prophylactic, diagnostic, or imaging active agents used in combination may be administered together in a single composition or separately in different compositions.

Those skilled in the art will recognize or be able to determine, using only routine experimentation, many equivalents to the specific embodiments described in this disclosure. The scope of this disclosure is not intended to be limited to the descriptions above, but rather as described in the appended claims.

In the claims, articles such as "a", "an" and "the" may refer to one or more than one, unless indicated otherwise or clearly evident from the context.

The term "comprising" is intended to be open-ended and allows for, but does not require, the inclusion of additional elements or steps. When the term "comprising" is used herein, the terms "consisting essentially of" and "consisting of" are therefore also covered and disclosed. Furthermore, it should be understood that the order of steps or the sequence of performing certain actions is not critical, as long as the disclosure remains operable. Two or more steps or actions may be performed simultaneously.

The compounds disclosed herein can be prepared in a variety of ways using commercially available starting materials, compounds known in the literature, or readily prepared intermediates, by employing standard synthetic methods and procedures known or to be known to those skilled in the art. Based on the teachings herein, the synthesis of the compounds disclosed is readily apparent to those skilled in the art. Standard synthetic methods and procedures for the preparation of organic molecules and for functional group transformations and manipulations can be obtained from relevant scientific literature or standard textbooks in the field, such as Smith, M. B., March, J., March's Advanced Organic Chemistry: Reactions, Mechanisms, and Structure, 5th edition, John Wiley & Sons: New York, 2001. The synthetic methods described in the examples herein are intended to illustrate, but not limit, the general procedures for preparing the compounds disclosed in this application. Those skilled in the art will appreciate that the order of certain steps in the reaction sequences and synthetic schemes described herein may be varied, such as the introduction and removal of protecting groups. Multiple stereoisomers may be produced in the reaction schemes described herein. Those skilled in the art will recognize that the reactions can be optimized to preferentially produce one isomer, or new schemes can be designed to produce a single isomer. If a mixture is produced, the isomers can be separated using techniques such as preparative thin-layer chromatography, preparative HPLC, preparative chiral HPLC, or preparative SFC.

In one or more embodiments, the compound of formula (I) described herein is a compound as shown below:

| | |
|---|---|
| **15** | 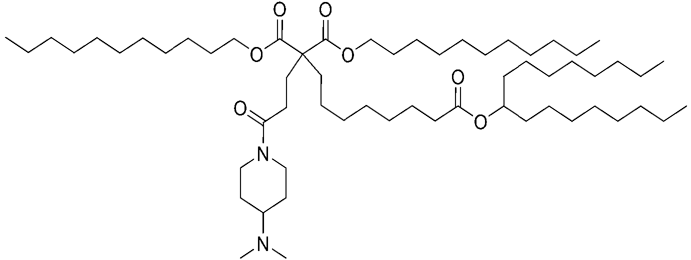 |
| **15A** | 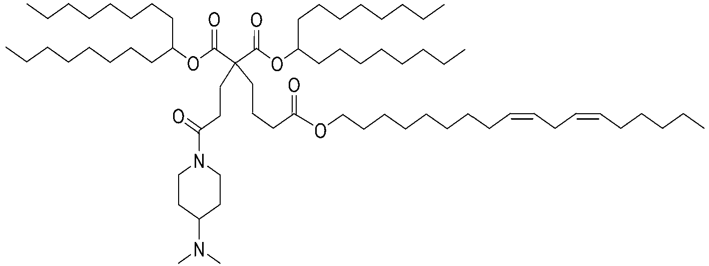 |
| **15B** | 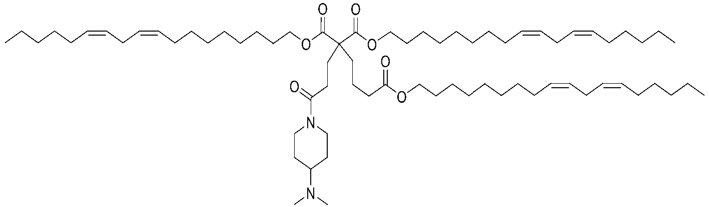 |
| **15C** | 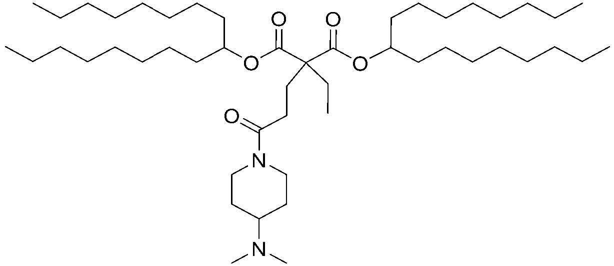 |
| **15D** | 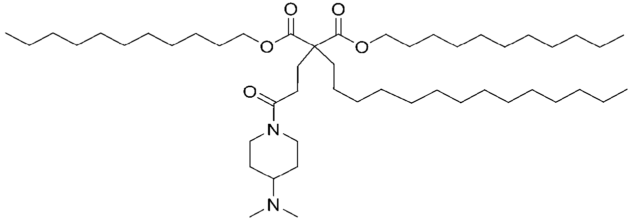 |
| **15E** | 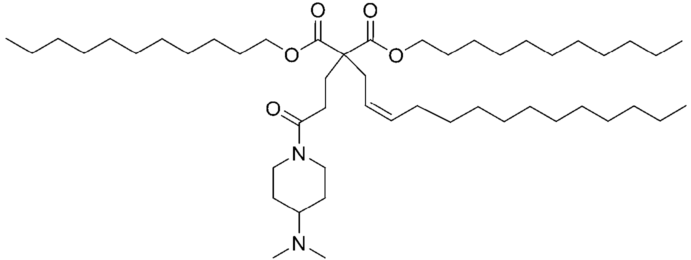 |
| **15F** | 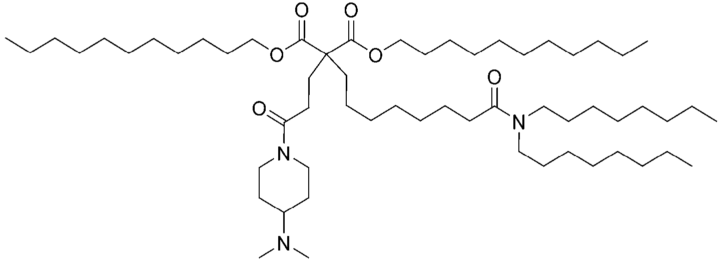 |

### EXAMPLE

### Example 1, Preparation of compounds

### Experimental instruments

| **Instruments** | **Model/Source** |
|---|---|
| Nuclear magnetic resonance spectrometer | Varian-600MHz |
| High resolution mass spectrometer | Thermo LTQ Orbitrap XL |

### Experimental reagents

| **Reagents** | **Source** |
|---|---|
| malonyl chloride | Energy Chemical |
| undecanol | Energy Chemical |
| 4-dimethylaminopyridine | Bide Pharmatech |
| sodium hydride | Energy Chemical |
| tetrabutylammonium iodide | Energy Chemical |
| triethylamine | Energy Chemical |

| toluene/methanol | Sinopharm Chemical Reagent Co.Ltd. |
|---|---|
| dichloromethane/petroleum ether/ethyl acetate | Energy Chemical |
| silica gel | Qingdao Ocean Chemical Co., Ltd |

### Synthesis process of compound 15:

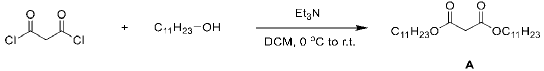

### Synthesis of Compound A (8-bromooctanoic acid heptadecane-9-yl ester)

A dry 100 mL two-necked flask equipped with a stir bar was charged with undecanol (20 mmol, 3.44 g) and dichloromethane (80 mL) under a nitrogen atmosphere. The reaction mixture was then placed in an ice bath. Subsequently, malonyl chloride (10 mmol, 1.40 g) and triethylamine (60 mmol, 6.06 g) were added to the reaction mixture, and the mixture was stirred at room temperature for 6 hours. After the reaction was complete, the organic solvent was removed using a rotary evaporator to obtain the crude product, which was then purified by column chromatography (petroleum ether/ethyl acetate = 20:1) to yield a colorless liquid, compound A (7.09 g, 86% yield).

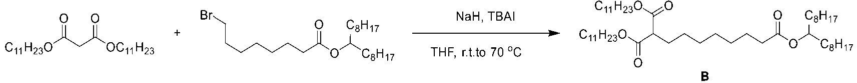

### Synthesis of Compound B (10-(heptadecan-9-yloxy)-10-oxo-2-[(undecyloxy)carbonyl] decanoic acid undecyl ester)

A dry 100 mL two-necked flask equipped with a stir bar was charged with tetrahydrofuran (15 mL), di(undecyl) malonate (3 mmol, 1.23 g), and NaH (2.6 mmol, 104 mg) under a nitrogen atmosphere. The mixture was stirred at room temperature for 30 minutes. Then, 8-bromooctanoic acid heptadecyl-9-yl ester (2 mmol, 0.92 g) and tetrabutylammonium iodide (0.4 mmol, 148 mg) were added under a nitrogen atmosphere, and the mixture was stirred at 70 °C for 48 hours. After the reaction was complete, the organic solvent was removed using a rotary evaporator to obtain the crude product, which was then purified by column chromatography (petroleum ether/ethyl acetate = 50:1) to yield a colorless liquid, compound B (0.84 g, 53% yield).

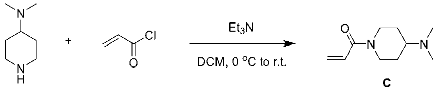

### Synthesis of Compound C (1-[4-(dimethylamino) hexahydropyridin-1-yl] prop-2-en-1-one)

A dry 50 mL two-necked flask equipped with a stir bar was charged with anhydrous dichloromethane (20 mL) and 4-dimethylaminopyridine (6.0 mmol, 768 mg) under a nitrogen atmosphere. The reaction mixture was cooled to 0 °C, and then acryloyl chloride (5 mmol, 455 mg) and triethylamine (7.5 mmol, 758 mg) were added sequentially. The mixture was stirred at room temperature for 8 hours. After the reaction was complete, the organic solvent was removed using a rotary evaporator to obtain the crude product, which was then purified by column chromatography (dichloromethane/methanol/triethylamine = 10:1:0.5) to yield a light yellow oily liquid, compound C (319 mg, 35% yield).

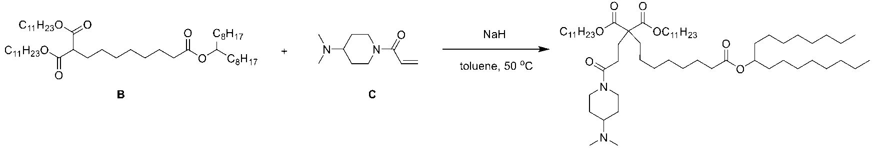

### Synthesis of Compound 15 (2-{3-[4-(dimethylamino) hexahydropyridin-1-yl]-3-oxopropyl}-10-(heptadecan-9-yloxy)-10-oxo-2-[(undecyloxy)carbonyl] decanoic acid undecyl ester)

A dry 25 mL Schlenk tube equipped with a stir bar was charged with toluene (1.5 mL), 10-(heptadecan-9-yloxy)-10-oxo-2-[(undecyloxy)carbonyl] decanoic acid undecyl ester (0.1 mmol, 79.2 mg), and NaOEt (0.03 mmol, 2 mg) under a nitrogen atmosphere. After stirring at room temperature for 10 minutes, 1-[4-(dimethylamino) hexahydropyridin-1-yl] prop-2-en-1-one (0.11 mmol, 20.1 mg) was added, and the mixture was stirred at 70 °C for 16 hours. After the reaction was complete, the organic solvent was removed using a rotary evaporator to obtain the crude product, which was then purified by column chromatography (dichloromethane/methanol = 20:1) to yield a colorless oily liquid, compound 15 (70.2 mg, 72% yield).**¹H NMR** (600 MHz, Chloroform-*d*) *δ* 4.85 (p, *J* = 6.3 Hz, 1H), 4.66 (d, *J* = 13.6 Hz, 1H), 4.16 - 4.01 (m, 4H), 3.92 (d, *J* = 13.8 Hz, 1H), 3.05 - 2.96 (m, 1H), 2.62 - 2.49 (m, 2H), 2.38 (s, 6H), 2.32 - 2.23 (m, 4H), 2.20 - 2.12 (m, 2H), 1.96 (d, *J* = 12.7 Hz, 1H), 1.92 - 1.82 (m, 3H), 1.64 - 1.55 (m, 6H), 1.50 (q, *J* = 6.1 Hz, 4H), 1.34 - 1.20 (m, 66H), 0.92 - 0.81 (m, 12H). **HRMS** (ESI) m/z (M+H)⁺ calculated for C60H115N2O7: 975.8704, observed: 975.8708.

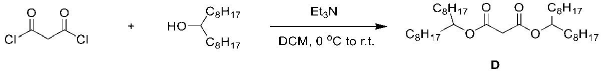

### Synthesis of Compound D (3-(heptadecan-9-yloxy)-3-oxopropanoic acid heptadecan-9-yl ester)

A dry 100 mL two-necked flask equipped with a stir bar was charged with 9-heptadecanol (20 mmol, 5.12 g) and dichloromethane (80 mL) under a nitrogen atmosphere. The reaction mixture was then placed in an ice bath. Subsequently, malonyl chloride (10 mmol, 1.40 g) and triethylamine (60 mmol, 6.06 g) were added to the reaction mixture, and the mixture was stirred at room temperature for 6 hours. After the reaction was complete, the organic solvent was removed using a rotary evaporator to obtain the crude product, which was then purified by column chromatography (petroleum ether/ethyl acetate = 20:1) to yield a colorless liquid, compound D (5.17 g, 89% yield).

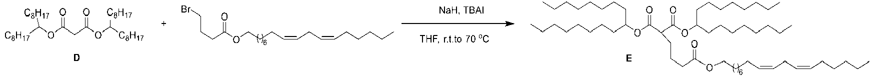

### Synthesis of Compound E (2-[(heptadecan-9-yloxy) carbonyl]-6-{[(10Z,12Z)-octadeca-9, 12-dienyl] oxy}-6-oxohexanoic acid heptadecan-9-yl ester)

A dry 100 mL two-necked flask equipped with a stir bar was charged with tetrahydrofuran (15 mL), di(9-heptadecyl) malonate (3 mmol, 1.74 g), and NaH (2.6 mmol, 104 mg) under a nitrogen atmosphere, and stirred at room temperature for 30 minutes. Then, 4-bromobutyric acid-(10*Z*,12*Z*)-octadeca-9,12-dien-1-yl ester (2 mmol, 0.83 g) and tetrabutylammonium iodide (0.4 mmol, 148 mg) were added under a nitrogen atmosphere, and the mixture was stirred at 70 °C for 48 hours. After the reaction was complete, the organic solvent was removed using a rotary evaporator to obtain the crude product, which was then purified by column chromatography (petroleum ether/ethyl acetate = 50:1) to yield a colorless liquid, compound E (1.06 g, 58% yield).

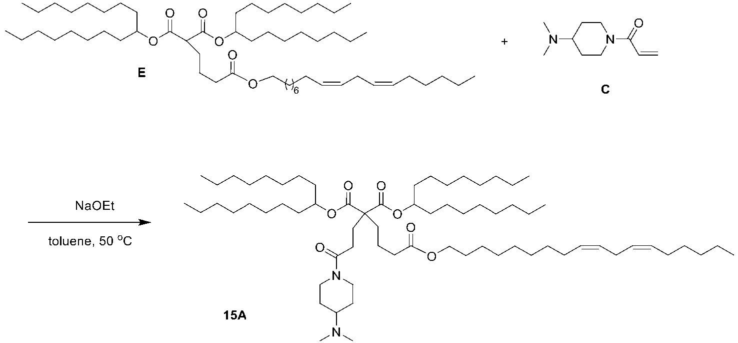

### Synthesis of Compound 15A (2-{3-[4-(dimethylamino) hexahydropyridine-1-yl]-3-oxopropyl}-2-[(heptadecan-9-yloxy) carbonyl]-6-{[(10Z,12Z)-octadeca-9,12-dienyl] oxy}-6-oxohexanoic acid heptadecan-9-yl ester)

A dry 25 mL Schlenk tube equipped with a a stir bar was charged with toluene (1.5 mL), compound E (0.1 mmol, 91.5 mg), and NaOEt (0.03 mmol, 2 mg) under a nitrogen atmosphere. After stirring at room temperature for 10 minutes, compound C (0.11 mmol, 20.1 mg) was added, and the mixture was stirred at 70 °C for 16 hours. After the reaction was complete, the organic solvent was removed using a rotary evaporator to obtain the crude product, which was then purified by column chromatography (dichloromethane/methanol = 20:1) to yield a colorless oily liquid, compound 15A (68.9 mg, 63% yield).**¹H NMR** (600 MHz, Chloroform-*d*) *δ* 5.42 - 5.24 (m, 4H), 4.86 (p, *J* = 6.3 Hz, 2H), 4.65 (d, *J* = 13.5 Hz, 1H), 4.02 (t, *J* = 6.9 Hz, 2H), 3.92 (d, *J* = 13.7 Hz, 1H), 2.97 (t, *J* = 12.8 Hz, 1H), 2.76 (t, *J* = 7.0 Hz, 2H), 2.57 - 2.51 (m, 1H), 2.34 (s, 6H), 2.32 - 2.26 (m, 4H), 2.21 (t, *J* = 8.3 Hz, 2H), 2.04 (q, *J* = 7.2 Hz, 4H), 1.94 - 1.84 (m, 4H), 1.62 - 1.56 (m, 2H), 1.55 - 1.46 (m, 8H), 1.35 - 1.20 (m, 69H), 0.87 (t, *J* = 7.0 Hz, 15H). **HRMS** (ESI) m/z (M+H)⁺ calculated for C₆₉H₁₂₉N₂O₇: 1097.9800, observed: 1097.9334.

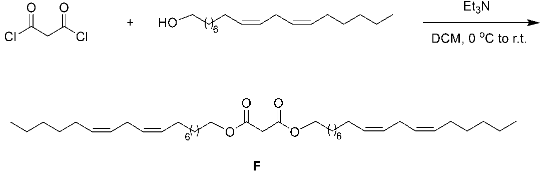

### Synthesis of Compound F (3-(heptadecan-9-yloxy)-3-oxopropanoic acid heptadecan-9-yl ester)

A dry 100 mL two-necked flask equipped with a stir bar was charged with (10*Z*,12*Z*)-octadeca-9,12-dien-1-ol (20 mmol, 5.32 g) and dichloromethane (80 mL) under a nitrogen atmosphere. The reaction mixture was then placed in an ice-water bath. Subsequently, malonyl chloride (10 mmol, 1.40 g) and triethylamine (60 mmol, 6.06 g) were added to the reaction mixture, and the mixture was stirred at room temperature for 6 hours. After the reaction was complete, the organic solvent was removed using a rotary evaporator to obtain the crude product, which was then purified by column chromatography (petroleum ether/ethyl acetate = 20:1) to yield a colorless liquid, compound F (4.96 g, 80% yield).

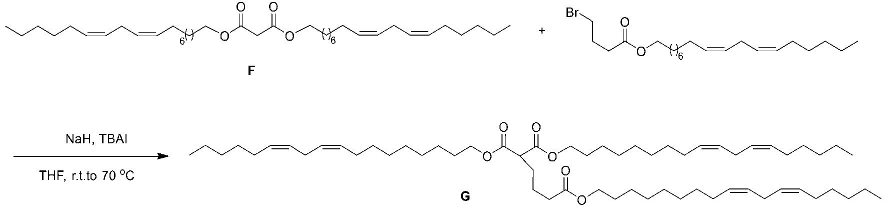

### Synthesis of Compound G (6-{[(10Z,12Z)-octadeca-9,12-dienyl]oxy}-2-({[(10Z,12Z)-octadeca-9,12-dienyl]oxy}carbonyl)-6-oxohexanoic acid (10Z,12Z)-octadeca-9,12-dien-1-yl ester)

A dry 100 mL two-necked flask equipped with a stir bar was charged with tetrahydrofuran (15 mL), Compound F (3 mmol, 1.80 g) and NaH (2.6 mmol, 104 mg) were added sequentially under nitrogen atmosphere, and the mixture was stirred at room temperature for 30 minutes. Then, 4-bromobutyric acid-(10*Z*,12*Z*)-octadeca-9,12-dien-1-yl ester (2 mmol, 0.83 g) and tetrabutylammonium iodide (0.4 mmol, 148 mg) were added under nitrogen atmosphere, and the mixture was stirred at 70 °C for 48 hours. After the reaction was complete, the organic solvent was removed using a rotary evaporator to obtain the crude product, which was then purified by column chromatography (petroleum ether/ethyl acetate = 50:1) to yield a colorless liquid, compound G (0.88 g, 47% yield).

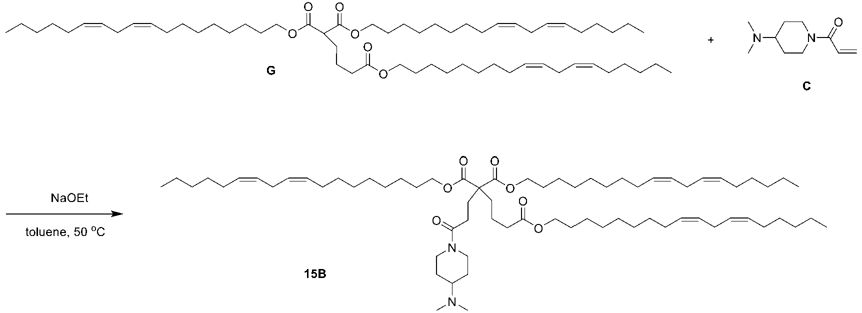

### Synthesis of Compound 15B (2-{3-[4-(dimethylamino) hexahydropyridin-1-yl]-3-oxopropyl}-6-{[(10Z,12Z)-octadeca-9,12-dienyl] oxy}-2-({[(10Z,12Z)-octadeca-9,12-dienyl] oxy} carbonyl)-6-oxohexanoic acid (10Z,12Z)-octadeca-9,12-dien-1-yl ester)

A 25mL dry Schlenk tube equipped with a stir bar was charged with toluene (1.5 mL), compound G (0.1 mmol, 93.5 mg), and NaOEt (0.03 mmol, 2 mg) under a nitrogen atmosphere. After stirring at room temperature for 10 minutes, compound C (0.11 mmol, 20.1 mg) was added, and the mixture was stirred at 70 °C for 16 hours. After the reaction was complete, the organic solvent was removed using a rotary evaporator to obtain the crude product, which was then purified by column chromatography (dichloromethane/methanol = 20:1) to yield a colorless oily liquid, compound 15B (60.3 mg, 54% yield).**¹H NMR** (600 MHz, Chloroform-*d*) *δ* 5.43 - 5.21 (m, 12H), 4.63 (d, *J* = 13.5 Hz, 1H), 4.14 - 4.05 (m, 4H), 4.03 (t, *J* = 6.9 Hz, 2H), 3.93 (d, *J* = 13.5 Hz, 1H), 2.99 (td, *J* = 13.0, 2.6 Hz, 1H), 2.76 (t, *J* = 7.0 Hz, 6H), 2.55 (td, *J* = 12.9, 2.8 Hz, 1H), 2.51 - 2.42 (m, 1H), 2.33 (s, 6H), 2.31 - 2.26 (m, 4H), 2.23 - 2.17 (m, 2H), 2.04 (q, *J* = 7.2 Hz, 12H), 1.93 - 1.83 (m, 4H), 1.65 - 1.57 (m, 6H), 1.56 - 1.49 (m, 2H), 1.37 - 1.23 (m, 50H), 0.88 (t, *J* = 6.9 Hz, 9H). **HRMS** (ESI) m/z (M+H)⁺ calculated for C₇₁H₁₂₅N₂O₇: 1117.9487, observed: 1117.9014.

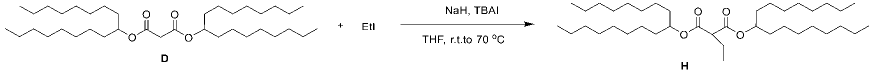

### Synthesis of Compound H (2-[(Heptadecan-9-yloxy)carbonyl] butanoic acid heptadecan-9-yl ester)

A dry 100 mL two-necked flask equipped with a stir bar was charged with tetrahydrofuran (15 mL), compound D (3 mmol, 1.74 g), and NaH (2.6 mmol, 104 mg) under a nitrogen atmosphere. The mixture was stirred at room temperature for 30 minutes. Then, iodoethane (2 mmol, 312 mg) and tetrabutylammonium iodide (0.4 mmol, 148 mg) were added under a nitrogen atmosphere, and the mixture was stirred at 70 °C for 48 hours. After the reaction was complete, the organic solvent was removed using a rotary evaporator to obtain the crude product, which was then purified by column chromatography (petroleum ether/ethyl acetate = 30:1) to yield a colorless liquid, compound H (0.83 g, 68% yield).

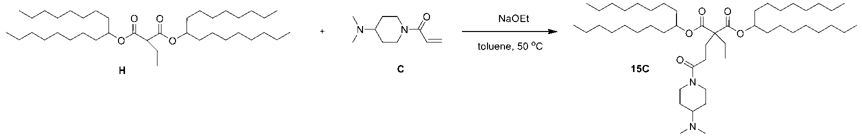

### Synthesis of Compound 15C (5-[4-(dimethylamino) hexahydropyridin-1-yl]-2-ethyl-2-[(heptadecan-9-yloxy) carbonyl]-5-oxopentanoic acid heptadecan-9-yl ester)

A 25mL dry Schlenk tube equipped with a stir bar was charged with toluene (1.5 mL), compound H (0.1 mmol, 60.9 mg), and NaOEt (0.03 mmol, 2 mg) under a nitrogen atmosphere. After stirring at room temperature for 10 minutes, compound C (0.11 mmol, 20.1 mg) was added, and the mixture was stirred at 70 °C for 16 hours. After the reaction was complete, the organic solvent was removed using a rotary evaporator to obtain the crude product, which was then purified by column chromatography (dichloromethane/methanol = 20:1) to yield a colorless oily liquid, compound 15C (50.3 mg, 71% yield). **¹H NMR** (600 MHz, Chloroform-*d*) *δ* 4.85 (p, *J* = 6.2 Hz, 2H), 4.65 - 4.53 (m, 1H), 3.93 - 3.79 (m, 1H), 2.94 (td, *J* = 13.0, 2.6 Hz, 1H), 2.52 (td, *J* = 12.9, 2.7 Hz, 1H), 2.38 (tt, *J* = 11.2, 3.7 Hz, 1H), 2.27 (s, 6H), 2.26 - 2.23 (m, 1H), 2.20 - 2.13 (m, 4H), 1.92 (q, *J* = 7.5 Hz, 2H), 1.87 - 1.78 (m, 2H), 1.54 - 1.43 (m, 8H), 1.33 - 1.14 (m, 52H), 0.85 (t, *J* = 7.0 Hz, 12H). **HRMS** (ESI) m/z (M+H)⁺ calculated for C₄₉H₉₅N₂O₅: 791.7241, observed: 791.6912.

### Example 2: Local expression activity testing of mRNA-LNP

### Experimental materials

| **Instrument/Reagent Name** | **Manufacturer** | **Product Number/Production Batch Number** |
|---|---|---|
| Compound 15 and its derivatives | self-prepared | - |
| SM-102 | AVT | SM220908 |
| PEG lipids | AVT | EDM210824 |
| Cholesterol | AVT | C00373 |
| DSPC | AVT | C20111 |
| Anhydrous ethanol | Sinopharm Chemical Reagent Co.Ltd. | 10009218 |
| Luc mRNA | Catug Biotechnology Co., Ltd. | P22J012 |
| DEPC-treated water | Biosharp | 22321483 |
| PBS | Wuhan Servicebio Technology Co., Ltd | G4202 |
| microfluidic instrument | Aitesen | MPE-L2 |
| PDMS chip | Aitesen | SP.000011 |
| isoflurane | RWD Life Science Co., Ltd | 21060901 |
| 10%TritonX-100 | Beyotime | ST797 |
| 3 M sodium acetate | Beyotime | ST351 |
| ultrafiltration tube | Millipore | UFC910096 |
| RNA quantification kit | ThermoFisher Scientific | R₁1490 |
| C57 mice | Shanghai SLAC Laboratory Animal Co, Ltd | - |
| SD rats | Shanghai SLAC Laboratory Animal Co, Ltd | - |
| precision syringe | BD | |
| in vivo substrate | Promega | P1043 |
| particle size analyzer | Malven | Nanozs |
| small animal in vivo imaging system | Perkin Elmer | IVIS Lumina K Series III |
| Spark Multifunctional Microplate Reader | TECAN | 30086376 |
| ultra-high-speed refrigerated centrifuge | ThermoFisher Scientific | 75005289 |

### Experimental methods

### 1. Preparation of LNP

The four components of the organic phase lipids were all dissolved in anhydrous ethanol to a concentration of 10 mg/mL, and then prepared according to the proportions given in Table 1; 1 mg/mL Luc mRNA was prepared in 25 mM sodium acetate buffer solution to a concentration of 70 µg/mL to serve as the aqueous phase; after cleaning the two-phase channels of the microfluidic device (stainless steel chip) with anhydrous ethanol and pure water respectively, the pipeline of the aqueous phase channel was rinsed twice with 25 mM sodium acetate buffer solution. At the start of preparation, the two-phase pipelines were purged of air with 300 µL of the prepared organic phase components and aqueous phase components respectively. Subsequently, the preparation solution with a volume ratio of organic phase to aqueous phase of 1:3 was aspirated, and LNPs were prepared at flow rates of 4 mL/min for the organic phase and 12 mL/min for the aqueous phase. The first 0.5 mL of the effluent was discarded, and only the subsequent effluent was collected until the end of the process (the preparation method for SM-102 LNP and 15 LNP was the same).

**Table 1**

| **Reagents** | Cationic lipid | PEG2000-DMG | Cholesterol | DSPC |
|---|---|---|---|---|
| SM-102 LNP-1 | 50 | 1.5 | 38.5 | 10 |
| SM-102 LNP-2 | 49.2 (50) | 3.0 (3.05) | 37.9 (38.5) | 9.8 (10) |
| 15 LNP-1 (The cationic lipid is Compound 15) | 50 | 1.5 | 38.5 | 10 |
| 15 LNP-2 (The cationic lipid is Compound 15) | 49.2 (50) | 3.0 (3.05) | 37.9 (38.5) | 9.8 (10) |
| 15 A (The cationic lipid is Compound 15A) | 49.2 (50) | 3.0 (3.05) | 37.9 (38.5) | 9.8 (10) |
| 15 B (The cationic lipid is Compound 15B) | 49.2 (50) | 3.0 (3.05) | 37.9 (38.5) | 9.8 (10) |
| 15 C (The cationic lipid is Compound 15C) | 49.2 (50) | 3.0 (3.05) | 37.9 (38.5) | 9.8 (10) |

| | | | | |
|---|---|---|---|---|
| Note: The unit is mole%, and the unit in parentheses is mole fraction. | | | | |

### 1.1 LNP ultrafiltration

The prepared LNP stock solution was added to 5 volumes of PBS. Ultrafiltration was performed using an ultrafiltration tube at 2000-3000 rpm for 10 minutes until the volume was approximately 1 mL. Then, 10 volumes of PBS were added to rinse the ultrafiltration membrane, followed by centrifugation until the volume was approximately 1 mL. 10 Volumes of PBS were added again to rinse the ultrafiltration membrane, followed by centrifugation until the volume was approximately 1 mL. The resulting 1 mL solution was transferred to an enzyme-free EP tube and stored at 4 °C for later use.

### 2. Determination of content and encapsulation efficiency

### 2.1 Preparation of standard curve

The mRNA stock solution was diluted with 1×TE to a concentration of 2 µg/mL. 0, 2, 10, 25, 50, and 100 µL of each concentration standard solution were added to a 96-well black microplate. The procedure was then followed according to the instructions of the RNA quantification kit (1×TE, 100 µL; 1×Ribo, 100 µL). The measurements were performed using a microplate reader with an excitation wavelength of 480 nm and an emission wavelength of 520 nm.

### 2.2 Preparation of LNP measurement samples

The ultrafiltered LNP were first diluted with 1×TE to a theoretical concentration of approximately 1 µg/mL. For permeabilized treatment, the diluted LNP were treated with a final concentration of 1% Triton X-100 at room temperature for 10 min. For nonpermeabilized treatment, the diluted LNP were incubated with an equal volume of DEPC-treated water at room temperature for 10 min. Then, 100 µL of each sample was transferred to a 96-well black microplate, followed by the addition of 100 µL of 1× Ribo solution to each well. Each sample was tested in triplicate. The fluorescence was measured using a microplate reader with an excitation wavelength of 480 nm and an emission wavelength of 520 nm.

### 3. Determination of particle size

An appropriate amount of ultrafiltered LNP was taken, diluted 10-fold with pure water, added into the particle size measurement cell, and then the particle size was determined using a Malvern particle size analyzer.

### 4. Local expression

Intramuscular Injection: Male C57 mice weighing approximately 20 g each were selected. Before injection, the hair on the gastrocnemius muscle of the lower leg was shaved first. Subsequently, a precision syringe was used to administer a dose of 50 µL of LNP (containing 10 µg of mRNA) into the gastrocnemius muscle per mouse. At 4 hours postadministration, 200 µL of luciferase substrate (15 mg/mL) was injected intraperitoneally. The mice were then anesthetized with isoflurane, followed by characterization using a small animal in vivo imaging system.

Intramyocardial injection: Male SD rats were anesthetized with isoflurane, and 40 µL of 15 LNP (12 µg mRNA) was injected into the myocardium of each rat via minimally invasive thoracotomy. Six hours after administration, 2 mL (15 mg/mL) of Luciferase substrate was injected intraperitoneally. Rats are anesthetized with isoflurane and subjected to in vivo imaging.

Spleen injection: Male C57 mice (approximately 20 g) were anesthetized with isoflurane, and the skin on the right side of the abdomen near the thoracic cavity was incised to expose the spleen. Then, a fine syringe was used to administer 20 µL of 15 LNP-2 (4 µg mRNA) per mouse into the spleen. Six hours after administration, 200 µL (15 mg/mL) of Luciferase substrate was injected intraperitoneally. The mice were then anesthetized with isoflurane for small animal in vivo imaging. Male SD rats (approximately 150 g) were injected with 30 µL (6 µg mRNA) each, and treated in the same method.

Kidney injection: Male C57 mice (approximately 20 g body weight) were anesthetized with isoflurane. The skin on the right side of the abdomen near the thoracic cavity was incised to expose the kidney. A fine syringe was then used to administer 20 µL of 15 LNP-2 (4 µg mRNA) into the kidney of each mouse. Six hours after administration, 200 µL (15 mg/mL) of Luciferase substrate was injected intraperitoneally. After isoflurane anesthesia, in vivo imaging was performed. Male SD rats (approximately 150 g body weight) were injected with 30 µL (6 µg mRNA) each, and treated in the same method.

### Experimental results

### 1. Particle size, PDI, encapsulation efficiency (%)

| LNP | encapsulation efficiency % | Size(d.nm) | PDI |
|---|---|---|---|
| SM-102 LNP-1 | 98 | 92.38 ± 1.639 | 0.035 ± 0.022 |
| SM-102 LNP-2 | 98 | 68.53 ± 1.003 | 0.053 ± 0.027 |
| 15 LNP-1 | 100 | 87.34 ± 2.507 | 0.054 ± 0.017 |
| 15 LNP-2 | 99 | 64.42 ± 0.7681 | 0.107 ± 0.037 |
| 15A | 98 | 73.84 ± 0.94 | 0.036 ± 0.028 |
| 15B | 98 | 74.50 ± 2.22 | 0.23 ± 0.02 |
| 15C | 97 | 64.57 ± 1.26 | 0.11 ± 0.04 |

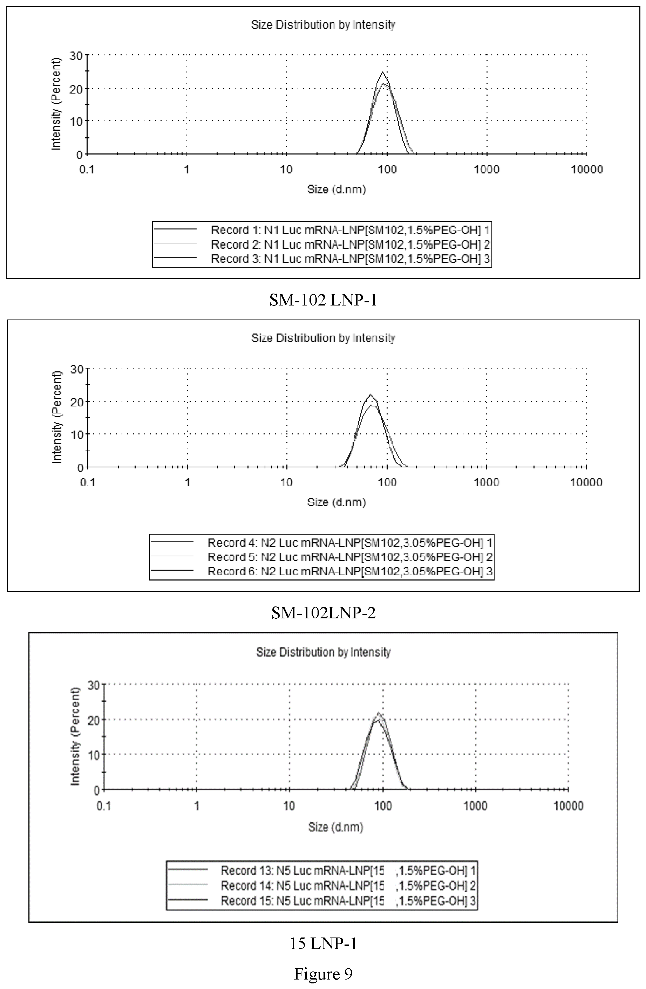

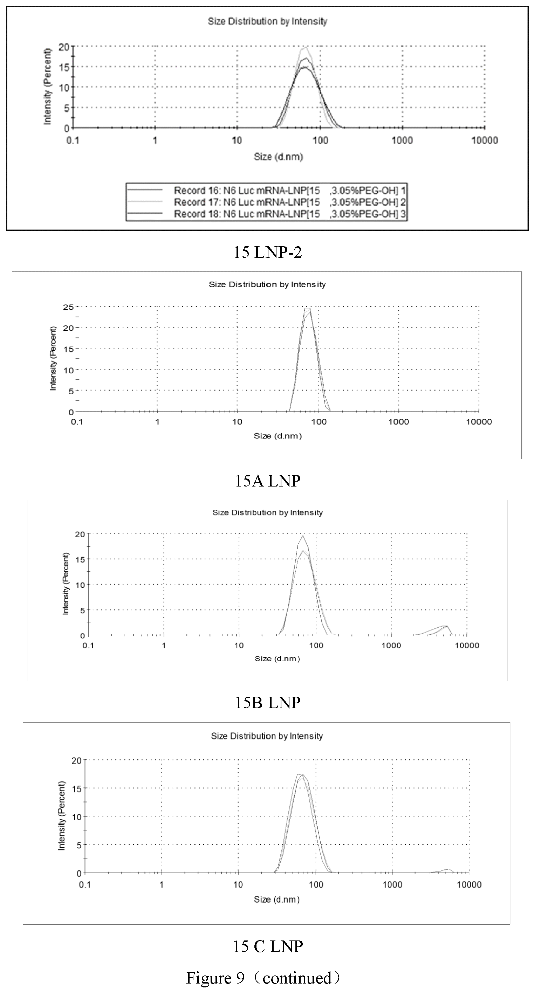

The particle size distribution of the LNP prepared from this compound is shown in Figure 9.

The particle size distribution of SM-102 is shown in Figure 9.

### 2. In vivo expression - Intramuscular injection

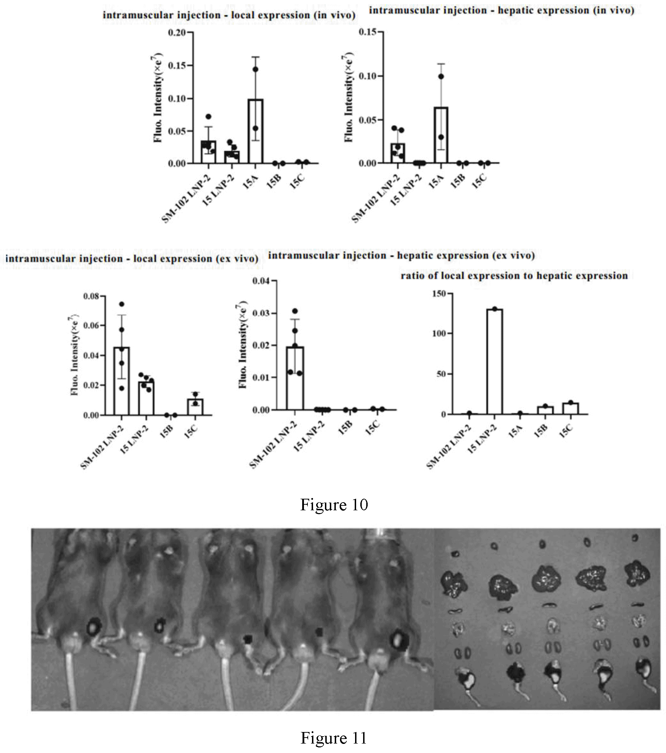

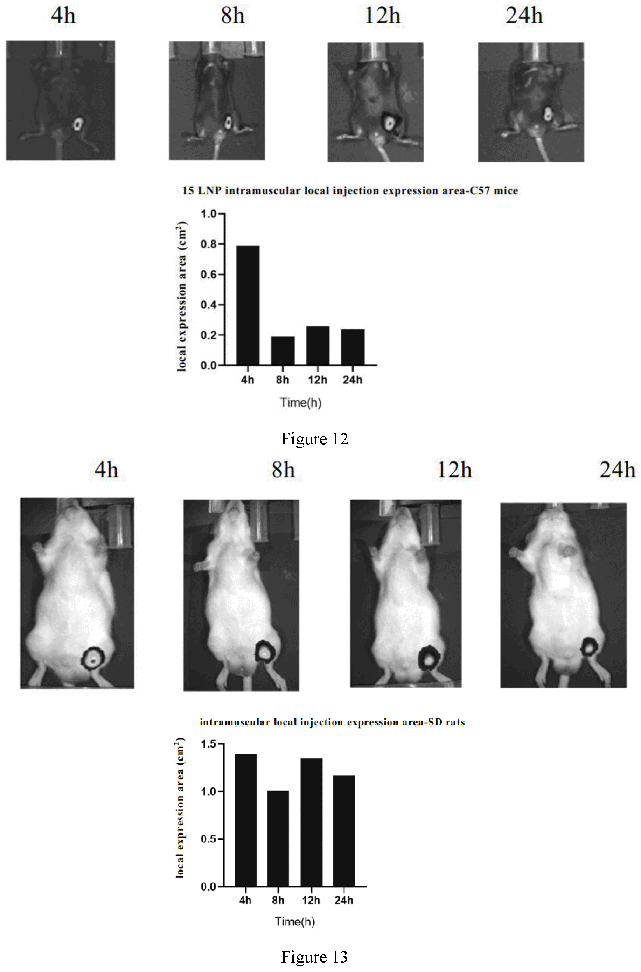

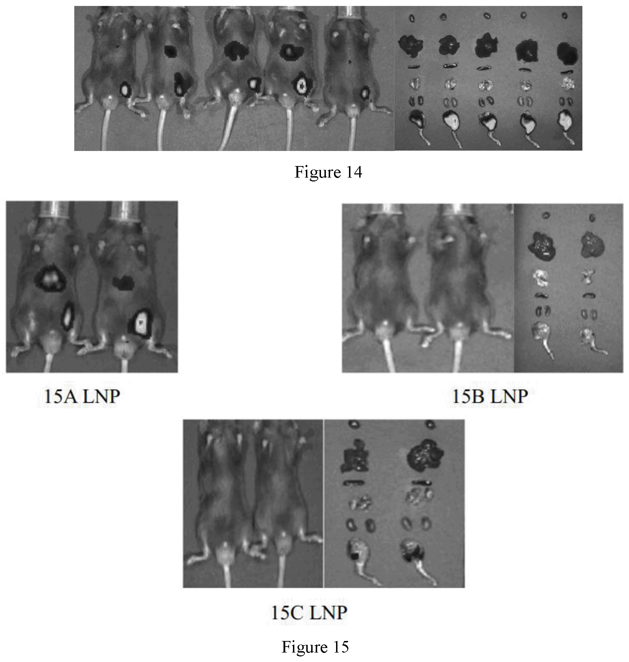

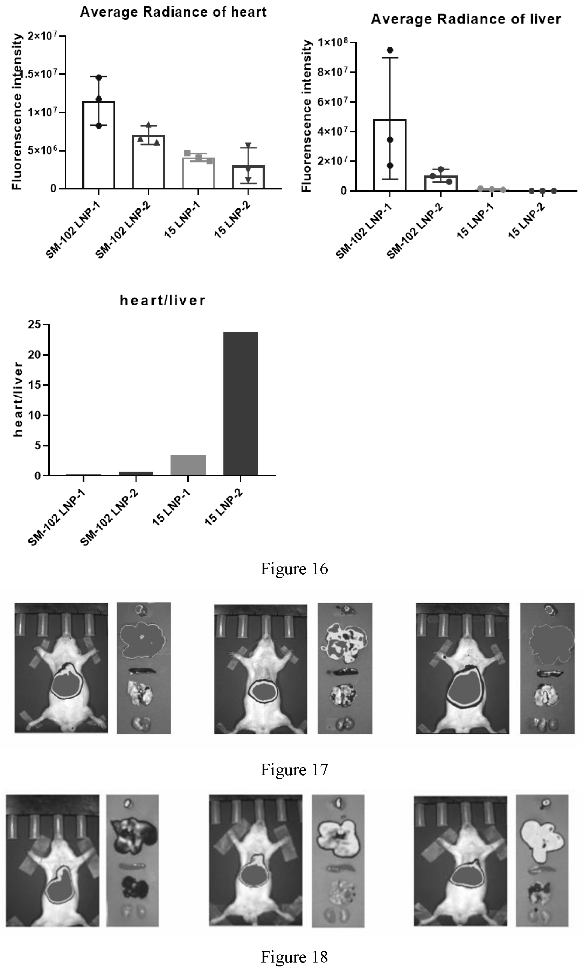

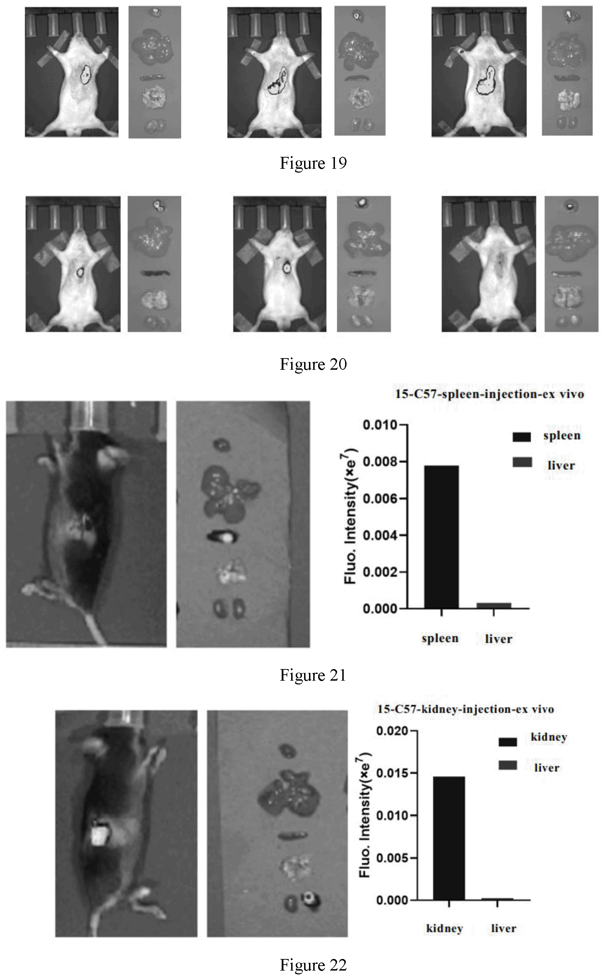

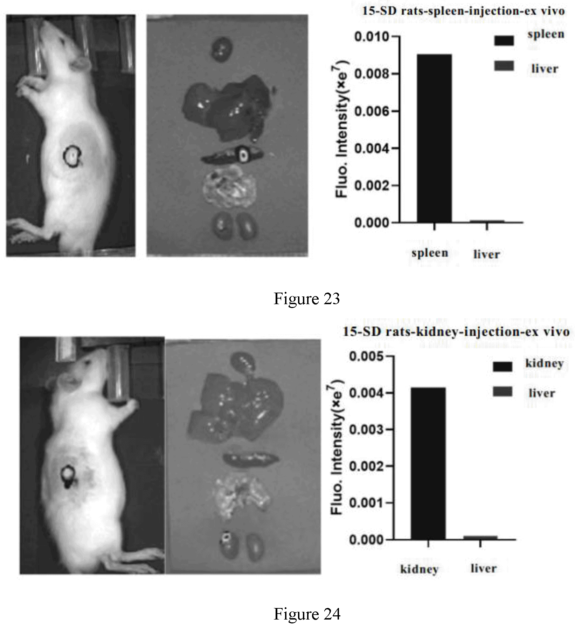

The results are shown in Figure 11-24. It can be seen that after intramuscular injection of 15 LNP-2, in vivo imaging of small animals showed expression only at the injection site, with no expression observed in other organs. After myocardial injection of 15 LNP-2, in vivo imaging of small animals showed expression only in the heart at the injection site, with no expression observed in other organs. After splenic injection of 15 LNP-2, in vivo imaging of small animals showed expression only in the spleen at the injection site, with no expression observed in other organs. After renal injection of 15 LNP-2, in vivo imaging of small animals showed expression only in the kidney at the injection site, with no expression observed in other organs.

## Claims

1. A compound of formula (I), or a salt or isomer thereof, 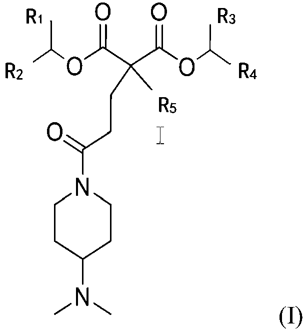 wherein,
R₁ is H or C1-C8 alkyl;
R₂ is C1-C10 alkyl, C3-C18 alkadienyl;
R₃ is H or R₁-R8 alkyl;
R₄ is C1-C10 alkyl or C3-C18 alkadienyl;
R₅ is C1-C14 alkyl, C2-C14 alkenyl, or 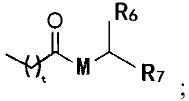
M is O or N;
t is an integer from 3 to 7;
R₆ is H or C1-C10 alkyl;
R₇ is C1-C10 alkyl or C3-C18 alkadienyl;
preferably, R₁ is identical to R₃, and R₂ is identical to R₄.

2. The compound or a salt or isomer thereof according to claim 1, wherein,
R₁ is H and R₂ is C6-C10 alkyl or C14-C18 alkadienyl, or R₁ is C4-C8 alkyl and R₂ is C4-C8 alkyl; and/or
R₃ is H and R₄ is C6-C10 alkyl or C14-C18 alkadienyl, or R₃ is C4-C8 alkyl and R₄ is C4-C8 alkyl.

3. The compound or a salt or isomer thereof according to claim 1 or 2, wherein, R₅ is C1-C4 alkyl, C10-C14 alkyl, C10-C14 alkenyl, or 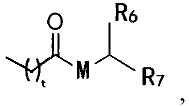 wherein,
M is O or N;
t is 3 or 7;
R₆ is C4-C8 alkyl and R₇ is C4-C8 alkyl, or R₆ is H and R₇ is C14-C18 alkadienyl.

4. A nanoparticle composition comprising a lipid component, wherein the lipid component comprises the compound or a salt or isomer thereof according to any one of claims 1 to 3,
preferably, the lipid component further comprises phospholipids, PEG lipids, structural lipids.

5. The nanoparticle composition according to claim 4, wherein the nanoparticle composition further comprises a therapeutic agent and/or a prophylactic agent.

6. A pharmaceutical composition comprising the nanoparticle composition according to claim 4 or 5, and pharmaceutically acceptable excipients.

7. A non-therapeutic method for producing a polypeptide in a cell comprising the step of contacting the cell with a nanoparticle composition, wherein the composition comprises: (1) a lipid component comprising phospholipids, PEG lipids, structural lipids, and the compound or a salt or isomer thereof according to any one of claims 1 to 3, and (2) an mRNA encoding the polypeptide, whereby the mRNA can be translated in the cell to produce the polypeptide,
preferably, the cell is a mammalian cell.

8. A method for preparing the compound or a salt or isomer thereof according to any one of claims 1 to 3, or for preparing the nanoparticle composition according to claim 4 or 5.

9. Use of the compound or a salt or isomer thereof according to any one of claims 1 to 3, and/or the nanoparticle composition according to claim 4 or 5, in the preparation of a medicament for treating or preventing a disease or disorder,
preferably, the medicament is a locally acting medicament,
preferably, the nanoparticle composition, or a nanoparticle composition formed from the compound of formula (I), is used for delivering a therapeutic agent and/or a prophylactic agent to a local region,
preferably, the disease or disorder is one that benefits from a therapeutic agent and/or a prophylactic agent for local delivery.

10. The use according to claim 9, wherein,
the local region is an organ, preferably heart, liver, spleen, kidney or lung; or
the local region is a tissue, preferably muscle or bone.
